# EUROPÄISCHE PATENTSCHRIFT

(11) **EP 0 493 711 B1**
(45) Veröffentlichungstag und Bekanntmachung des Hinweises auf die Patenterteilung: **25.09.1996**
(21) Anmeldenummer: 91121148.0
(22) Anmeldetag: 10.12.1991
(51) Int. Cl.: C07C 251/48, C07C 59/68, C07C 255/40, C07C 65/21, C07C 235/34

(54) **Verfahren zur Herstellung von E-Oximethern von Phenylglyoxylsäureestern**
Process for the preparation of E-oximethers from phenylglyoxylic acid esters
Procédé de préparation d'oximéthers d'esters de l'acide phénylglyoxylique

(30) Priorität: 31.12.1990 DE 4042273; 31.12.1990 DE 4042280; 31.12.1990 DE 4042282; 31.12.1990 DE 4042283; 31.12.1990 DE 4042271; 31.12.1990 DE 4042272
(43) Veröffentlichungstag der Anmeldung: 08.07.1992
(60) Teilanmeldung: 96101025.3
(73) Patentinhaber: BASF Aktiengesellschaft, 67063 Ludwigshafen (DE)
(72) Erfinder: Wingert, Horst, Dr., W-6800 Mannheim 1 (DE); Wolf, Bernd, Dr., W-6701 Fussgoenheim (DE); Benoit, Remy, Dr., W-6700 Ludwigshafen (DE); Sauter, Hubert, Dr., W-6800 Mannheim 1 (DE); Hepp, Michael, Dr., W-6802 Ladenburg (DE); Grammenos, Wassilios, Dr., W-6700 Ludwigshafen (DE); Kuekenhoehner, Thomas, Dr., W-6710 Frankenthal (DE)

(56) Entgegenhaltungen:
- EP-A- 0 253 213
- EP-A- 0 398 692
- EP-A- 0 400 417
- DE-B- 1 279 682

## Beschreibung

Die vorliegende Erfindung betrifft ein Verfahren zur Herstellung von E-Oximethern von Phenylglyoxylsäureestern der allgemeinen Formel I 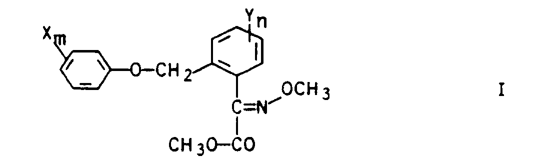 wobei die Variablen die folgende Bedeutung haben:
- X,Y: Substituenten, ausgewählt aus einer Gruppe bestehend aus Halogen, C₁-C₄-Alkyl, C₁-C₄-Alkoxy oder Trifluormethyl;
- m: eine ganze Zahl von 0 bis 4;
- n: eine ganze Zahl von 0 bis 3.

Es ist bekannt, Oximether vom Typ der Verbindungen I durch Umsetzung von Glyoxylsäureestern mit O-Methylhydroxylaminhydrochlorid herzustellen (vgl. z.B. EP-A 253 213 und EP-A 254 426), wobei als Nebenprodukt äquimolare Mengen an Chlorwasserstoff gebildet werden. Nachteilig bei diesem Verfahren ist jedoch, daß E/Z-Isomerengemische der Oximether entstehen, die nur mit relativ großem Aufwand technisch getrennt werden können. Dabei erhält man das Isomere mit der bevorzugten E-Konfiguration an der Oximbindung oftmals nur in sehr geringen Mengen.

Aufgabe der vorliegenden Erfindung war es deshalb, die Verbindungen I besser zugänglich zu machen.

Demgemäß wurde ein Verfahren zur Herstellung von E-Oximethern von Phenylglyoxylsäureestern I gefunden, welches dadurch gekennzeichnet ist, daß man ein o-Phenoxymethylbenzoylcyanid der allgemeinen Formel VI 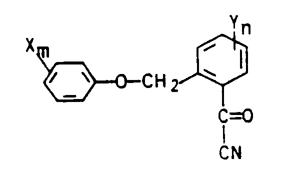 in Methanol in Gegenwart einer Säure in ein Gemisch enthaltend die Derivate VIIa und VIIb 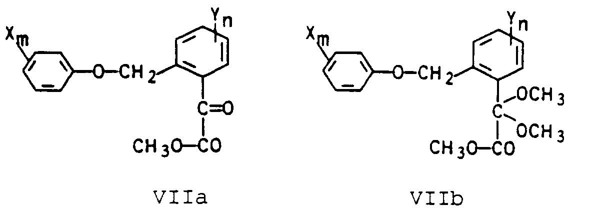 überführt und dieses Gemisch anschließend mit O-Methylhydroxylamin oder einem seiner Säureadditionssalze umsetzt und gleichzeitig oder anschließend mit einer Säure behandelt.

Die Phenylglyoxylsäureester der Formel VIIa sind beispielsweise nach dem folgenden Verfahren erhältlich:

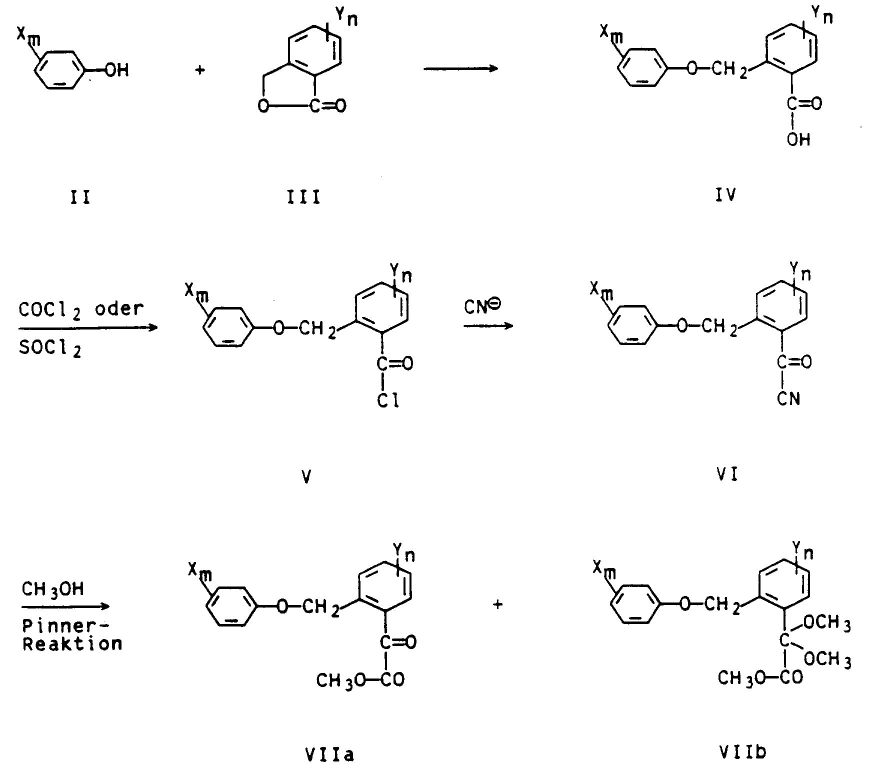

Im ersten Verfahrensschritt werden Phenole der Formel II mit Lactonen der Formel III, bevorzugt unter basischen Bedingungen, umgesetzt [vgl. z.B. Czech. Chem. Commun. 32, 3448 (1967)]. Die erhaltenen o-(Phenoxymethyl)-benzoesäuren IV überführt man zweckmäßigerweise in ihre Säurechloride V (vgl. Organikum, VEB Deutscher Verlag der Wissenschaften, 16. Auflage, Berlin 1986, Seite 423 f.) und stellt aus diesen dann die entsprechenden Benzoylcyanide VI her.

Die Benzoylcyanide VI werden anschließend einer Pinner-Reaktion unterworfen [(vgl. Angew. Chemie 94, 1 (1982)], wobei sowohl die Phenylglyoxylsäureester VIIa als auch, als Nebenprodukte, die Ketale der Phenylglyoxylsäureester VIIb entstehen. Diese können in an sich bekannter Weise in die Verbindungen VIIa überführt werden.

Sowohl die Phenylglyoxylsäureester VIIa als auch die Ketale der Phenylglyoxylsäureester VIIb oder Gemische dieser Verbindungen können nach dem erfindungsgemäßen Verfahren in die E-Oximether der Phenylglyoxylsäureester I überführt werden. Insbesondere kann das durch die Pinner Reaktion erhaltene Rohprodukt-Gemisch aus Phenylglyoxylsäureester VIIa und Ketal des Phenylglyoxylsäureesters VIIb ohne weitere Reinigung nach diesem Verfahren in die E-Oximether der Phenylglyoxylsäureester I überführt werden.

Das O-Methylhydroxylamin wird entweder in Form eines Säureadditionssalzes oder als freie Base eingesetzt, wobei die unprotonierte Verbindung durch Zugabe einer starken Base aus dem Salz freigesetzt werden kann. Als Salze der O-Methylhydroxylamine kommen die Salze mit ein- bis dreiwertigen Säuren, wie insbesondere Salzsäure und Schwefelsäure, in Betracht.

Im allgemeinen nimmt man die Reaktion in Gegenwart eines Lösungs- oder Verdünnungsmittels, vor.

Geeignete Lösungsmittel sind vorzugsweise aromatische Kohlenwasserstoffe wie Benzol, Toluol und o-, m-, p-Xylol, chlorierte Kohlenwasserstoffe wie Methylenchlorid, Alkohole wie Methanol und Ether wie Diethylether. Besonders bevorzugt ist Methanol.

Die Mengenverhältnisse der Edukte sind nicht kritisch; zweckmäßigerweise setzt man stöchiometrische Mengen an Ausgangsverbindungen ein, sofern sich nicht ein Überschuß der einen oder anderen Komponente, z.B. 10 mol-%, empfiehlt.

Normalerweise liegt die Reaktionstemperatur zwischen 0 und 100°C, vorzugsweise zwischen 20 und 80°C.

Eine Verfahrensvariante besteht darin, die aus der Pinner-Reaktion erhaltene Mischung aus den Verbindungen VIIa und VIIb ohne Isolierung aus der Reaktionsmischung mit O-Methylhydroxylamin oder einem seiner Säureadditionssalze umzusetzen.

Die Oximether von Phenylglyoxylsäureestern werden in der Regel als Isomerengemische erhalten, wobei die Oximbindung teilweise in der E- und teilweise in der Z-Konfiguration vorliegt. Die Umlagerung der Oximether in die E-Konfiguration erfolgt durch Behandlung mit einer Säure.

Zu diesem Zweck kann die Rohlösung zuvor eingeengt oder weiter verdünnt werden. Gewünschtenfalls kann die Umlagerung auch in einem 2-Phasensystem aus Wasser/Säure und einem organischen Lösungsmittel wie Dichlormethan erfolgen. Zweckmäßig behandelt man die erhaltene Rohlösung des Oximethers jedoch ohne weitere Aufkonzentration oder Verdünnung direkt mit der Säure.

Als Säuren eignen sich insbesondere Mineralsäuren, beispielsweise Perchlorsäure, Schwefelsäure, Phosphorsäure und Halogenwasserstoffsäuren wie Chlorwasserstoff, aliphatische Sulfonsäuren wie Trifluormethansulfonsäure, aromatische Sulfonsäuren wie p-Toluolsulfonsäure sowie halogenierte Alkancarbonsäuren wie Trifluoressigsäure. Besonders bevorzugt ist Chlorwasserstoff-Gas.

Üblicherweise verwendet man die 1- bis 20-fache, insbesondere die 3- bis 10-fache, molare Menge an Säure, bezogen auf die Menge an VIIa bzw. VIIa und VIIb.

Die Reaktionstemperatur für die Isomerisierung liegt im allgemeinen zwischen (-20) und 100°C, insbesondere zwischen 20 und 80°C.

Die Umlagerung der Oximether benötigt eine gewisse Zeit und zwar abhängig von der Temperatur und insbesondere der Säuremenge etwa 1 bis 90 Stunden, vorzugsweise 4 bis 20 Stunden.

In der Regel können alle genannten Verfahrensschritte bei Atmosphärendruck oder unter dem Eigendruck des jeweiligen Systems, bis ca. 5 bar, ausgeführt werden. Höherer oder niedrigerer Druck ist auch möglich, bietet im allgemeinen jedoch keine Vorteile.

Das erfindungsgemäße Verfahren kann sowohl diskontinuierlich als auch kontinuierlich durchgeführt werden. Bei der kontinuierlichen Arbeitsweise leitet man die Reaktionspartner beispielsweise durch einen Rohrreaktor oder über Rührkesselkaskaden.

Das erfindungsgemäße Verfahren läßt sich mit Erfolg zur Synthese aller definitionsgemäßen E-Oximethern von Phenylglyoxylsäureestern anwenden, vor allem auf solche Verbindungen, in denen die Substituenten X und Y unabhängig voneinander die folgende Bedeutung haben:
- Halogen wie Fluor, Chlor und Brom;
- verzweigtes oder unverzweigtes C₁-C₄-Alkyl wie Methyl, Ethyl, Isopropyl und n-Butyl, insbesondere Methyl und Ethyl;
- C₁-C₄-Alkoxy wie Methoxy, Ethoxy, 1-Methylethoxy und n-Propoxy;
- Trifluormethyl.

Die E-Oximether von Phenylglyoxylsäureestern I werden nach dem erfindungsgemäßen Verfahren überraschenderweise in hoher Ausbeute und ausgezeichneter Reinheit zugänglich. Im Hinblick auf den bekannten Stand der Technik war dagegen zu erwarten, daß bei dem Verfahren die gleichen Schwierigkeiten auftreten, wie bei den bereits bekannten Herstellverfahren. Insbesondere war keinesfalls vorauszusehen, daß unter sauren Bedingungen eine Isomerisierung mit überwiegender Bildung des E-Isomeren stattfindet, da bei den bekannten Synthesemethoden durch den Einsatz von O-Methylhydroxylaminhydrochlorid bereits äquimolare Mengen an Chlorwasserstoff entstehen, ohne daß ein Isomeres bevorzugt entsteht. Weiterhin hätte man bei Behandlung der Rohprodukte mit Säure Zersetzungsreaktionen des Oximethers erwartet.

Das erfindungsgemäße Verfahren besitzt eine Reihe von Vorteilen:
- es ist in technischem Maßstab in sehr einfacher Weise durchführbar;
- die Salze des O-Methylhydroxylamins können als wässrige Lösungen eingesetzt werden;
- der Phenylglyoxylsäureester VIIa kann als Rohprodukt aus der Vorstufe eingesetzt werden, da das als Verunreinigung enthaltene Dimethylketal des Phenylglyoxylsäureesters überraschenderweise auch in den gewünschten Oximether überführt wird.

Die E-Oximether von Phenylglyoxylsäureestern I werden in der EP-A 253 213 und der EP-A 254 426 als Pflanzenschutzmittel beschrieben.

### Beispiel 1

### E-2-(Phenoxymethyl)-phenylglyoxylsäuremethylester-O-methyloxim

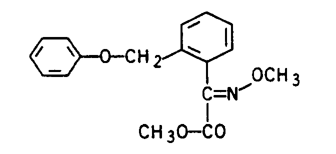

Eine Mischung aus 6,1 g (22,5 mmol) 2-(Phenoxymethyl)-phenylglyoxylsäuremethylester, 2,1 g (25 mmol) O-Methylhydroxylaminhydrochlorid und 40 ml Methanol wurde 9 Stunden auf Rückflußtemperatur erhitzt. Dann entfernte man das Lösungsmittel bei reduziertem Druck, versetzte den Rückstand mit 100 ml Methylenchlorid und leitete bei 20°C bis zur Sättigung Chlorwasserstoff-Gas ein. Nach 12 Stunden Rühren bei 20°C wurde die organische Lösung mit Wasser gewaschen, getrocknet und eingeengt. Das bräunliche Rohprodukt kristallisierte beim Anreiben mit kalten Methanol. Ausbeute: 6,3 g.

¹H-NMR (in CDCl₃; TMS als interner Standard): 7,55 ppm (d,1H), 7,40 ppm (m,2H), 7,25 ppm (m,3H), 6,90 ppm (m,3H), 4,95 ppm (s,2H), 4,00 ppm (s,3H), 3,85 ppm (s,3H).

### Vorstufe 1.1

### 2-(Phenoxymethyl)-benzoesäure

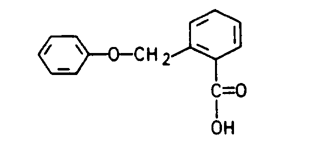

Eine Mischung aus 151 g (1,6 mol) Phenol, 106 g 85 gew.-%ige wässrige Kaliumhydroxid-Lösung (entsprechend 1,6 mol KOH) und 1,5 l Xylol wurde unter laufender Entfernung des Wassers auf Rückflußtemperatur erhitzt. Nach Entfernung des Wassers versetzte man das Reaktionsgemisch bei 100°C mit 201 g (1,5 mol) Phthalid und 57 ml Dimethylformamid und rührte anschließend noch 15 Stunden bei dieser Temperatur. Nach dem Abkühlen auf 20-25°C wurde das Produktgemisch zweimal mit je 2 l Wasser extrahiert und dann mit 140 ml 38 gew.-%iger wässriger Salzsäure versetzt. Die gebildeten Kristalle wurden abgetrennt, mit 500 ml Wasser gewaschen und getrocknet. Zur Reinigung löste man das Rohprodukt in 550 ml warmem Aceton und fällte es durch Zugabe von 3 l Wasser wieder aus. Ausbeute: 296 g; Fp.: 125-127°C;

¹H-NMR (in CDCl₃, TMS als interner Standard): 5,55 ppm (s,2H); 7,00 ppm (m,3H); 7,30 ppm (t,2H); 7,40 ppm (t,1H); 7,65 ppm (t,1H); 7,85 ppm (d,1H); 8,20 ppm (d,1H).

### Vorstufe 1.2

### 2-Phenoxymethylbenzoylchlorid

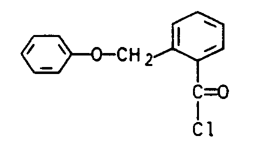

Eine Mischung aus 72 g (0,32 mol) 2-Phenoxymethylbenzoesäure, 56 g (0,47 mol) Thionylchlorid und 300 ml 1,2-Dichlorethan wurde 3 Stunden auf Rückflußtemperatur erhitzt. Nach Entfernen der flüchtigen Bestandteile im Vakuum erhielt man ein dunkles Öl, das langsam auskristallisierte. Ausbeute: 79 g.

¹H-NMR (in CDCl₃, TMS als interner Standard): 5,35 ppm (s,2H); 6,95 ppm (m,3H); 7,3 ppm (t,2H); 7,5 ppm (t,1H); 7,7 ppm (t,1H); 7,85 ppm (d,1H); 8,35 ppm (d,1H).

### Vorstufe 1.3

### 2-Phenoxymethylbenzoylcyanid

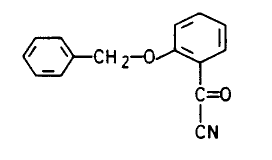

Bei 20°C wurde eine Mischung aus 79 g (0,32 mol) 2-Phenoxymethylbenzoylchlorid, 17 g (0,347 mol) Natriumcyanid, 0,3 g (0,93 mmol) Tetrabutylammoniumbromid, 200 ml Wasser und 300 ml 1,2-Dichlorethan 2 Stunden kräftig gerührt. Anschließend trennte man die Phasen und extrahierte die wässrige Phase mit 100 ml 1,2-Dichlorethan. Die vereinigten organischen Phasen wurden dreimal mit je 100 ml Wasser gewaschen und dann getrocknet. Nach Entfernen des Lösungsmittels unter reduziertem Druck erhielt man 70,8 g eines bräunlichen Öles, das beim Anreiben mit Methyl-tert.-butylether/Pentan (1:1) kristallisierte.

¹H-NMR (in CDCl₃, TMS als interner Standard): 8,40 ppm (d,1H); 8,00 ppm (d,1H); 7,80 ppm (t,1H); 7,60 ppm (t,1H); 7,35 ppm (t,2H); 7,00 ppm (m,3H); 5,45 ppm (s,2H).

### Vorstufe 1.4

### 2-(Phenoxymethyl)-phenylglyoxylsäuremethylester

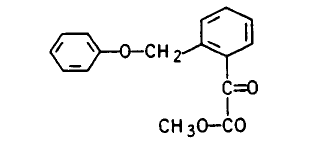

In einer Mischung aus 10 g (42 mmol) 2-(Phenoxymethyl)-benzoylcyanid, 4,3 g (42 mmol) Acetanhydrid und 50 ml Methyl-tert.-butylether wurde unter Rühren bei 0 bis (-5)°C bis zur Sättigung Chlorwasserstoffgas eingeleitet. Nach 10 Stunden Rühren bei 20°C erhielt man eine Lösung, die mit 50 ml Methanol versetzt wurde. Man erhitzte noch 10 Stunden auf Rückflußtemperatur und entfernte anschließend unter reduziertem Druck leichtflüchtige Bestandteile, wonach der Rückstand in 150 ml Methyl-tert.-butylether gelöst wurde. Die organische Phase wurde zweimal mit je 100 ml Wasser gewaschen, dann getrocknet und eingeengt. Man erhielt eine ölige Mischung aus 68 % des gewünschten Produktes und 32 % des entsprechenden Dimethylketals. Zur Spaltung des Dimethylketals wurde die Mischung, gelöst in 50 ml Dichlormethan, mit 10 ml konzentrierter Salzsäure 10 Stunden bei 20 bis 25°C gerührt. Danach wurde die organische Phase abgetrennt, dreimal mit je 50 ml Wasser gewaschen, getrocknet und eingeengt. Ausbeute: 9,7 g (rötliches Öl).

¹H-NMR (in CDCl₃, TMS als Standard): 7,75 ppm (m,2H), 7,60 ppm (t,1H), 7,45 ppm (t,1H), 7,30 ppm (m,2H), 6,95 ppm (m,3H), 5,40 ppm (s,2H) und 3,80 ppm (s,3H).

### Beispiel 2

### E-2-(2'-Methylphenoxymethyl)-phenylglyoxylsäuremethylester-O-methyloxim

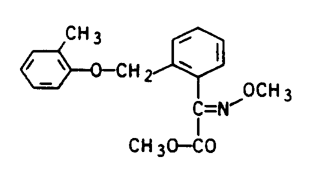

### Variante 2.1

129 g eines Gemisches, das zu 80 Gew.-% aus 2-(2'-Methylphenoxymethyl)-phenylglyoxylsäuremethylester und zu 13 Gew.-% aus 2-(2'-Methylphenoxymethyl)-phenylglyoxylsäuremethylester-dimethylketal bestand, wurden zusammen mit 41,7 g (0,5 mol) O-Methylhydroxylaminhydrochlorid und 450 ml Methanol 7 Stunden auf Rückflußtemperatur erhitzt. Nach Abkühlen auf 20°C versetzte man die Reaktionsmischung mit 450 ml Methylenchlorid und leitete 219 g (6 mol) Chlorwasserstoff-Gas ein. Man rührte noch 15 Stunden bei dieser Temperatur und entfernte anschließend das Lösungsmittel bei reduziertem Druck. Der Rückstand wurde zuerst mit kaltem Methanol und dann mit Petrolether gewaschen und anschließend getrocknet. Ausbeute: 106,8 g (farbloser Feststoff).

### Variante 2.2

Eine Mischung aus 56,8 g 2-(2'-Methylphenoxymethyl)-phenylglyoxylsäuremethylester (90 % rein; 0,18 mol), 55,7 g einer 30 %igen wässrigen O-Methylhydroxylaminhydrochlorid-Lösung (= 0,20 mol) und 200 ml Methanol wurde 7 Stunden auf Rückflußtemperatur erhitzt. Anschließend leitete man bei 0 bis 10°C 72 g (1,97 mol) Chlorwasserstoffgas in die Mischung, versetzte sie mit 200 ml Methylenchlorid und rührte noch drei Tage bei 20 bis 25°C. Nach Abdestillieren des Lösungsmittels bei reduziertem Druck wurde der Rückstand mit kaltem Methanol und Petrolether gewaschen und dann getrocknet. Ausbeute: 50,5 g

### Variante 2.3

In eine Mischung aus 753 g (3 mol) 2-(2'-Methylphenoxymethyl)-benzoylcyanid, 336,6 g (3,3 mol) Acetanhydrid und 2,3 l Methyl-tert.-butylether wurden bei (-18) bis (-8)°C 1343 g (36,8 mol) Chlorwasserstoff-Gas eingeleitet. Anschließend rührte man 36 Stunden bei 20 bis 25°C, versetzte das Reaktionsgemisch mit 1,8 l Methanol und erhitzte noch 15 Stunden auf Rückflußtemperatur. Nach Kühlen auf 20 bis 25°C entfernte man das Lösungsmittel bei reduziertem Druck und versetzte den Rückstand mit 1 l Methylenchlorid. Die organische Phase wurde erst mit 800 ml Wasser gewaschen, dann mit 800 ml konzentrierter wässriger Salzsäure versetzt und 15 Stunden bei 20 bis 25°C gerührt. Nach Phasentrennung wurde die wässrige Phase noch mit 0,5 l Methylenchlorid gewaschen. Die vereinigten organischen Phasen wurden eingeengt.

Anschließend versetzte man das Rohprodukt (Hauptanteil: 2-(2'-Methylphenoxymethyl)-phenylglyoxylsäuremethylester) mit 2 l Methanol und 250,5 g (3 mol) O-Methylhydroxylaminhydrochlorid und erhitzte dieses Gemisch 15 Stunden auf Rückflußtemperatur. Zu dem Reaktionsgemisch wurden bei 20 bis 25°C noch 2 l Methylenchlorid gegeben. Dann leitete man 986 g (27 mol) Chlorwasserstoff-Gas ein und rührte weitere 48 Stunden. Nach Abdestillieren des Lösungsmittels bei reduziertem Druck wurde der Rückstand mit Methanol, Wasser und Petrolether gewaschen und dann getrocknet. Gesamtausbeute: 814 g.

### Variante 2.4

Eine Mischung aus 57,9 g (0,2 mol) 2-(2'-Methylphenoxymethyl)-phenylglyoxylsäuremethylester, 18,4 g (0,22 mol) O-Methylhydroxylaminhydrochlorid und 135 ml Methanol wurde 6 Stunden auf Rückflußtemperatur erhitzt. Anschließend leitete man bei 20°C 36,5 g (1,0 mol) Chlorwasserstoffgas in die Mischung ein und rührte 24 Stunden bei 20°C. Der ausgefallene Feststoff wurde abgesaugt, mit 100 ml kaltem Methanol gewaschen und getrocknet. Ausbeute: 57,2 g.

Fp.: 97 - 98°C.

¹H-NMR (in CDCl)₃; TMS als interner Standard): 2,22 ppm (3H), 3,8 ppm (3H), 4,02 ppm (3H), 4,94 ppm (2H), 6,8 ppm (2H), 7,11 ppm (2H), 7,2 ppm (1H), 7,4 ppm (2H), 7,57 ppm (1H).

Die Benzoylcyanide VI sind auf verschiedene Weise erhältlich. Üblicherweise werden sie aus den entsprechenden Säurechloriden und cyanidhaltigen Salzen wie Quecksilbercyanid [Liebig's Annalen 3, 249 (1832)], Kupfercyanid [Organic Synthesis Coll. Vol. III, 112 (1955); Angew. Chemie 94(1), 1 (1982)] und insbesondere Natriumcyanid unter Verwendung eines wässrigorganischen Zweiphasensystems und in Gegenwart eines Phasentransfer katalysators [Tetrahedron Letters, 2275 (1974)] hergestellt:

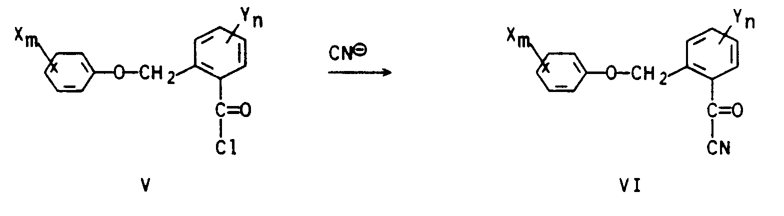

Die Umsetzung erfolgt normalerweise in einem inerten Lösungsmittel oder Lösungsmittelgemisch, wobei das cyanidhaltige Salz bevorzugt in Wasser gelöst und das Säurechlorid V in einem mit Wasser mischbaren oder unmischbaren Lösungsmittel gelöst vorliegt.

Besonders bevorzugt arbeitet man in einem Zweiphasensystem in Gegenwart eines Phasentransferkatalysators, z.B. einem quartären Ammonium- oder Phosphoniumsalz wie Tetrabutylammoniumchlorid, Tetrabutylammoniumbromid, Methyltributylammoniumjodid, Tetrabutylammoniumhydrogensulfat, Trimethylbenzylammoniumchlorid, Triethylbenzylammoniumchlorid, Triphenylbenzylammoniumchlorid und Benzyltributylphosphoniumbromid.

Als cyanidhaltige Salze eignen sich bevorzugt Alkalimetallcyanide wie Lithium-, Natrium- und Kaliumcyanid oder Erdalkalimetallcyanide wie Magnesium-, Calcium- oder Bariumcyanid, insbesondere Natrium- und Kaliumcyanid. Es können aber auch Mischungen der genannten Salze mit Blausäure verwendet werden.

Als inerte Lösungs- oder Verdünnungsmittel für die Säurechloride V eignen sich beispielweise aliphatische Kohlenwasserstoffe wie n-Pentan, n-Hexan, Cyclohexan, Petrolether, Weißöle und Ligroin, aromatische Kohlenwasserstoffe wie Benzol, Toluol und o-, m-, p-Xylol, halogenierte Kohlenwasserstoffe wie Methylenchlorid, Chloroform, Tetrachlorkohlenstoff, 1,2-Dichlorethan, 1,1,2-Trichlorethan, 1,1,2,2-Tetrachlorethan, Perchlorethan und Chlorbenzol, Ether wie Diethylether, Methyl-tert.-butylether, Tetrahydrofuran und Dioxan, Ester wie Ethylacetat, Amide wie Dimethylformamid sowie Dimethylsulfoxid, Sulfolan und N-Methylpyrrolidon.

Normalerweise werden das cyanidhaltige Salz oder die Mischung eines cyanidhaltigen Salzes in Blausäure und das Säurechlorid V in stöchiometrischen Mengen eingesetzt, jedoch kann auch ein Überschuß an cyanidhaltigem Salz und gegebenenfalls Blausäure, bis etwa 100 mol-%, vorteilhaft sein.

Die Menge an Phasentransferkatalysator ist nicht kritisch; üblicherweise verwendet man 0,001 bis 1 mol-%, insbesondere 0,01 bis 5 mol-% an Katalysator, bezogen auf die Menge an V.

Zweckmäßigerweise arbeitet man bei Normaldruck. Höherer oder niedrigerer Druck sind möglich, bringen jedoch im allgemeinen keine Vorteile.

Die Reaktionstemperatur liegt im allgemeinen zwischen 0°C und der Siedetemperatur des jeweiligen Lösungsmittel(gemisches), insbesondere zwischen 0 und 40°C.

Die Aufarbeitung des Reaktionsgemisches erfolgt nach an sich bekannten Methoden, so daß sich nähere Ausführungen hierzu erübrigen.

Die Umsetzung kann sowohl diskontinuierlich als auch kontinuierlich durchgeführt werden. Bei der kontinuierlichen Arbeitsweise leitet man die Reaktionspartner beispielsweise durch einen Rohrreaktor oder über Rührkesselkaskaden.

Eine bevorzugte Ausführungsvariante besteht darin, das cyanidhaltige Salz oder die Mischung eines cyanidhaltigen Salzes in Blausäure zusammen mit dem Phasentransferkatalysator vorzulegen und eine Lösung des Säurechlorids unter intensivem Rühren zuzugeben.

2-(Phenoxymethyl)-benzoesäurechlorid (V; m, n = 0) ist aus der DE-A 1 279 682 bekannt.

Des weiteren sind aus der JP-OS 80/124 777 einige durch Alkylgruppen substituierte 2-(Phenoxymethyl)-benzoesäurechloride als Zwischenprodukte für 6,11-Dihydro-11-oxodibenz(b,e)oxepine bekannt.

Die o-substituierten Benzoylchloride der allgemeinen Formel V' 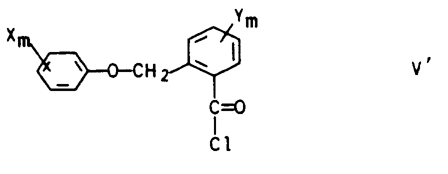 wobei die Variablen die folgende Bedeutung haben:
- X,Y: Halogen, C₁-C₄-Alkyl, C₁-C₄-Alkoxy oder Trifluormethyl;
- m: eine ganze Zahl von 0 bis 4;
- n: eine ganze Zahl von 0 bis 3,

ausgenommen 2-(Phenoxymethyl)-benzoesäurechlorid,
2-(3'-n-Butylphenoxymethyl)-benzoesäurechlorid,
2-(3'-tert.-Butylphenoxymethyl)-benzoesäurechlorid,
2-(3',4'-Dimethylphenoxymethyl)-benzoesäurechlorid,
2-(4'-sec.-Butylphenoxymethyl)-benzoesäurechlorid und
2-(4'-Ethylphenoxymethyl)-4-(methoxy)-benzoesäurechlorid, sind neu.

Sie können nach an sich bekannten Verfahren durch Behandlung der entsprechenden Carbonsäuren IV mit Phosgen oder Thionylchlorid, zweckmäßigerweise in einem inerten Lösungs- oder Verdünnungsmittel, erhalten werden (vgl. Organikum, VEB Deutscher Verlag der Wissenschaften, 16. Auflage, Berlin 1986, Seiten 423 f).

Als Lösungs- oder Verdünnungsmittel eignen sich beispielsweise aliphatische Kohlenwasserstoffe wie n-Pentan, n-Hexan, Cyclohexan, Petrolether, Weißöle und Ligroin, aromatische Kohlenwasserstoffe wie Benzol, Toluol und o-, m-, p-Xylol, halogenierte Kohlenwasserstoffe wie Methylenchlorid, Chloroform, Tetrachlorkohlenstoff, 1,2-Dichlorethan, 1,1,2-Trichlorethan, 1,1,2,2-Tetrachlorethan, Perchlorethan und Chlorbenzol, Ether wie Diethylether, Tetrahydrofuran und Dioxan, Ester wie Ethylacetat sowie Ketone wie Aceton, Methylethylketon und Cyclohexanon.

Carbonsäure IV und Chlorierungsmittel werden normalerweise in stöchiometrischen Mengen eingesetzt, bevorzugt verwendet man einen Überschuß an Chlorierungsmittel bis zu 100 mol-%.

Vorteilhaft arbeitet man in Anwesenheit eines Katalysators, beispielsweise eines Amides wie Dimethylformamid, Diethylformamid und Diisobutylformamid, eines tertiären Amins wie Dimethylaminopyridin, Diethylaminopyridin, Morpholinoformiat und 4-Pyrrolidinopyridin oder eines Phosphinoxides wie Tributylphosphinoxid.

Die Menge an Katalysator kann in weiten Bereichen variiert werden. Üblicherweise verwendet man 0,01 bis 50 mol-%, insbesondere 0,1 bis 10 mol-% an Katalysator, bezogen auf die Menge an Säure IV.

Die Reaktionstemperatur ist nicht kritisch. Im allgemeinen arbeitet man zwischen 0 und 100°C, insbesondere bei der Siedetemperatur des jeweiligen Lösungsmittels.

Zweckmäßigerweise erfolgt auch die Herstellung der Säurechloride unter Normaldruck. Höherer oder niedrigerer Druck sind möglich, bringen jedoch im allgemeinen keine Vorteile.

Die Aufarbeitung des Reaktionsgemisches erfolgt wie üblich.

Die Umsetzung kann sowohl diskontinuierlich als auch kontinuierlich durchgeführt werden. Bei der kontinuierlichen Arbeitsweise leitet man die Reaktionspartner beispielsweise durch einen Rohrreaktor oder über Rührkesselkaskaden.

Eine Verfahrensvariante zur Herstellung der Benzoylcyanide VI besteht darin, die Benzoylchloride V zu synthetisieren und sie ohne Isolierung aus der Reaktionsmischung oder als Rohprodukte mit Alkalimetall- oder Erdalkalimetallcyaniden, gewünschtenfalls in Gegenwart von Blausäure, zu behandeln.

Die Carbonsäuren IV sind entweder aus den Druckschriften US-A 3 420 851, JP-OS 80/124 777 oder aus Coll. Czech. Chem. Commun. 32, 3448 (1967) bekannt oder nach den dort beschriebenen Methoden herstellbar. Besonders vorteilhaft stellt man sie durch Umsetzung von Phenolen der Formel II mit Lactonen der Formel III, bevorzugt unter basischen Reaktionsbedingungen, her.

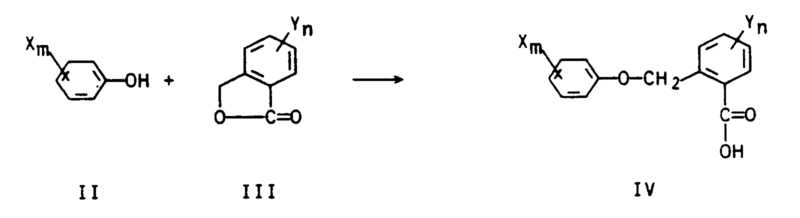

Die O-substituierten Benzoylcyanide VI sind wertvolle Zwischenprodukte für die Synthese von E-Oximethern von Phenylglyoxylsäureestern I 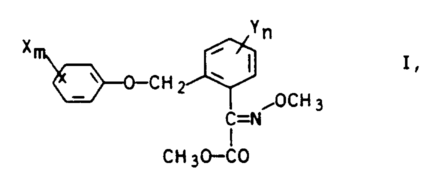 die im Pflanzenschutz vorzugsweise als Fungizide Verwendung finden (vgl. EP-A 253 213 und EP-A 254 426).

Zur Herstellung der Endprodukte I werden die O-substituierten Benzoylcyanide VI, entweder in isolierter Form, als Rohprodukte oder noch in dem bei ihrer Herstellung verwendeten Lösungsmittel gelöst, mit niederen Alkoholen wie Methanol nach der Pinner-Reaktion (vgl. Angew. Chemie 94, 1 (1982) umgesetzt. Dabei erhält man sowohl Phenylglyoxylsäureester VIIa als auch die Ketale der Phenylglyoxylsäureester VIIb. Anschließend setzt man die Verbindungen VIIa oder VIIb oder ein Gemisch aus beiden mit O-Methylhydroxylamin oder einem seiner Säureadditionssalze um, wobei das hierbei erhaltenen E/Z-Isomerengemisch zur weitgehenden Umlagerung der Z-in die E-Isomeren gleichzeitig oder anschließend mit einer Säure behandelt wird:

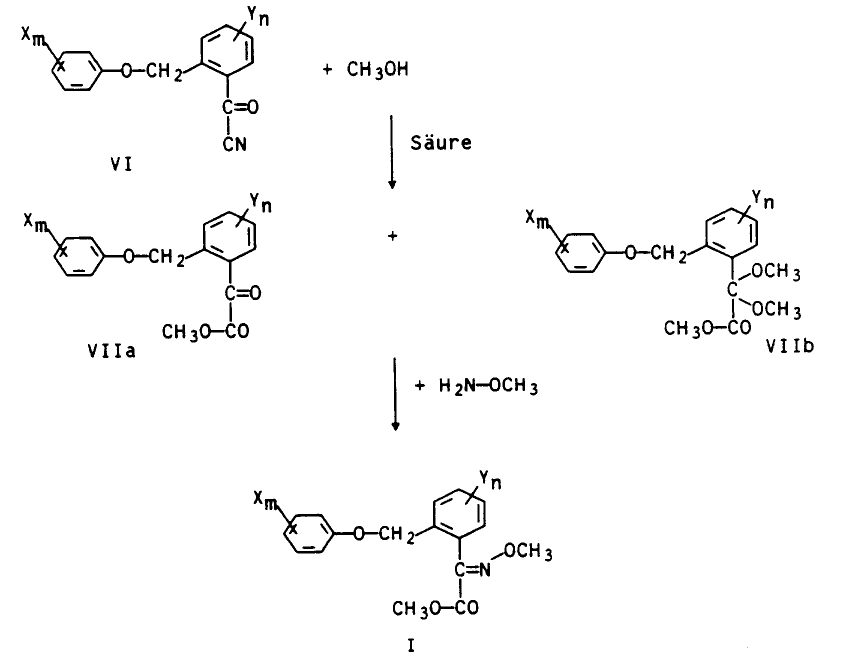

### Beispiel 5

### 2-(2'-Methylphenoxymethyl)benzoylcyanid

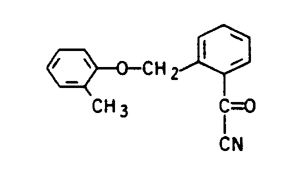

### Variante A:

Zu einer Lösung von 458 g (1,76 mol) 2-(2'-Methylphenoxymethyl)-benzoylchlorid in 2 l Toluol wurden 890 mg (2,8 mmol) Tetra-n-butylammoniumbromid und anschließend eine Lösung von 94,8 g (1,93 mol) Natriumcyanid in 1 l Wasser gegeben. Das Gemisch wurde 2 Stunden bei etwa 20°C gerührt, und dann 14 Stunden stehen gelassen. Anschließend wurde die organische Phase abgetrennt, über Natriumsulfat getrocknet und bei reduziertem Druck eingeengt. Das braune Rohprodukt mit einer Reinheit von 91,7 gew.-% (nach GC-Analyse) wurde aus 2 l Methyl-tert.-Butylether umkristallisiert. Ausbeute: 61 %;

### Variante B:

Zu einer Mischung aus 48,2 g (0,98 mol) Natriumcyanid, 2,5 g (7,5 mmol) Tetra-n-butylammoniumchlorid und 640 ml Wasser wurde bei 22°C innerhalb einer Stunde eine Lösung von 231,8 g (0,89 mol) 2-(2'-Methylphenoxymethyl)-benzoylchlorid in 1,55 l Toluol zugetropft. Man rührte noch 14 Stunden, wonach die organische Phase abgetrennt, einmal mit 0,5 l Wasser gewaschen und bei reduziertem Druck eingeengt wurde. Das Rohprodukt wurde aus 0,4 l Cyclohexan umkristallisiert. Ausbeute: 67 %.

Fp.: 74°C (gelb-brauner Feststoff).

### Variante C: (Reaktionsführung in Gegenwart von Blausäure)

Zu einer Lösung von 117,4 g (2,4 mol) Natriumcyanid in 725 ml Wasser wurden bei 20°C 38 g einer 19 gew.-%igen wäßrigen Salzsäure (= 0,2 mol HCl) getropft. Nach Zugabe von 1,4 g (4,3 mmol) Tetrabutylammoniumbromid und 2,4 l Toluol wurde das Gemisch auf 25°C erwärmt und unter intensivem Rühren innerhalb von 15 Minuten mit einer Lösung von 521 g (2,0 mol) 2-(2'-Methylphenoxymethyl)-benzoylchlorid in 710 ml Toluol versetzt. Man rührte noch 90 Minuten, wobei sich die Reaktionsmischung bis 32°C erwärmte. Anschließend wurde die organische Phase abgetrennt, mit 200 ml Wasser und 200 ml 1 gew.-%iger wäßriger Salzsäure gewaschen und dann unter reduziertem Druck eingeengt. Das blaßgelbe Rohprodukt enthielt 93 mol-% 2-(2'-Methylphenoxymethyl)-benzoylcyanid (nach HPLC-Analyse). Ausbeute: 92,5 %.

¹H-NMR (in CDCl₃, TMS als interner Standard): 2,3 ppm (s,3H); 5,4 ppm (s,2H); 6,7-7,0 ppm (m,2H); 7,1-7,3 ppm (m,2H); 7,6 ppm, (t,1H); 7,8 ppm (t,1H); 8,0 ppm (d,1H); 8,3 ppm m (d,1H);

IR-Spektrum [cm-1]: 2220, 1665, 1600, 1573, 1496, 1242, 1131, 1060, 746, 724.

### Vorstufe 5.1

### 2-(2'-Methylphenoxymethyl)benzoylchlorid

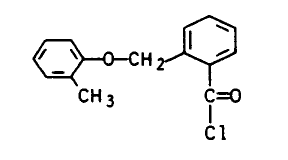

Zu einer Lösung von 435 g (1,80 mol) 2-(2'-Methylphenoxymethyl)-benzoesäure in 1,8 l Toluol wurden bei 20 bis 25°C 235,6 g (1,98 mol) Thionylchlorid getropft. Die erhaltene Mischung wurde auf 70°C erhitzt, bis keine Gasentwicklung mehr zu beobachten war (ca. 5 Stunden) und dann eingeengt. Das erhaltene braune Öl (Gehalt an Produkt: 92,5 %) kristallisierte bei Abkühlung. Ausbeute: 93,5 %; Fp.: 48 - 51°C.

¹H-NMR (in CDCl₃; TMS als interner Standard): 2,3 ppm (s,3H), 5,3 ppm (s,2H), 6,7-6,9 ppm (m,2H), 7,0-7,2 ppm (m,2H), 7,4 ppm (t,1H), 7,6 ppm (t,1H), 7,9 ppm (d,1H), 8,3 ppm (d,1H);

IR-Spektrum [cm⁻¹]: 1737, 1495, 1243, 1026, 1185, 1124, 886, 743, 716, 673.

Die Erfindung betrifft ferner ein neues Verfahren zur Herstellung von α-Ketocarbonsäureestern der allgemeinen Formel VIIa 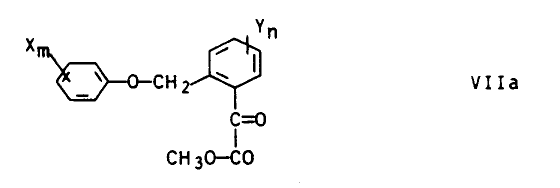 wobei die Variablen die folgende Bedeutung haben:
- X,Y: Substituenten, ausgewählt aus einer Gruppe bestehend aus Halogen, C₁-C₄-Alkyl, C₁-C₄-Alkoxy oder Trifluormethyl;
- m: eine ganze Zahl von 0 bis 4;
- n: eine ganze Zahl von 0 bis 3.

In der Literatur werden verschiedene Verfahren zur Darstellung aromatischer α-Ketoester aus den entsprechenden Benzoylcyaniden beschrieben.

In den Druckschriften Angew. Chemie 68, 430 (1956) und dto. 94, 1 (1982), Org. Synth. 24, 16 (1944) sowie J. Org. Chem. 29, 278 (1964) werden Methoden beschrieben, Benzoylcyanide mit konzentrierten Mineralsäuren zu hydrolysieren und die entstehenden Ketocarbonsäuren in Phenylglyoxylsäureester zu überführen, wobei jedoch Benzoesäureester als Nebenprodukte gebildet werden:

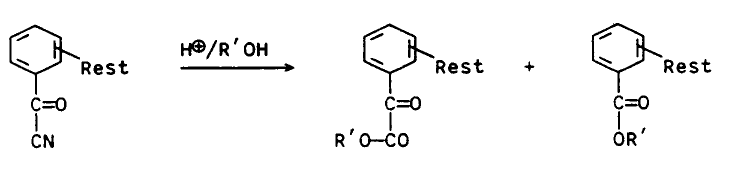

Gemäß der aus US-A 4 234 739, DE-A 2 708 189 und Tetrah. Lett., 3539 (1980) bekannten Lehre kann die Bildung der Benzoesäureester in mehreren Fällen durch Halogenid-Zusatz weitgehend unterdrückt werden.

Außerdem ist bekannt, Benzoylcyanide entweder in die entsprechenden tert.-Butylketoamide IX nach der Ritter-Reaktion (vgl. EP-A 034 240) oder in N-Acylketoamide X (vgl. EP-A 35 707) zu überführen. Beide Verbindungen können anschließend zu Ketocarbonsäuren und Ketoestern umgesetzt werden:

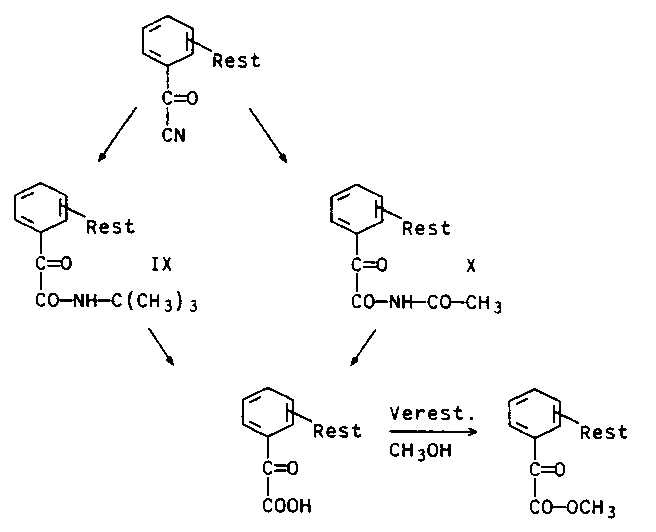

Nach den genannten Methoden sind die α-Ketocarbonsäureester VIIa aus den Benzoylcyaniden VI jedoch nicht herstellbar. Insbesondere wird hierzu in der Angew. Chem. 68, 430 (1956) und dto. 94, 10 (1982) gelehrt, daß die Durchführung der Pinner-Reaktion mit Benzoylcyaniden bei Raumtemperatur in einem sauren Medium, in Gegenwart eines Alkohols, zu den entsprechenden Benzoesäureestern und nicht zu Ketocarbonsäureestern führt.

Der vorliegenden Erfindung lag daher die Aufgabe zugrunde, ein Verfahren zur Herstellung der Verbindungen VIIa aus den Benzoylcyaniden VI bereitzustellen.

Bei der Umsetzung der Benzoylcyanide gemäß dem erfindungsgemäßen Verfahren können als Nebenprodukte die entsprechenden Ketocarbonsäureester-Dimethylketale VIIb der Zwischenprodukte VIIa entstehen. Diese Nebenprodukte sind jedoch für die weitere Verwendung der α-Ketocarbonsäureester VIIa zur Synthese von Phenylglyoxylsäureestern I 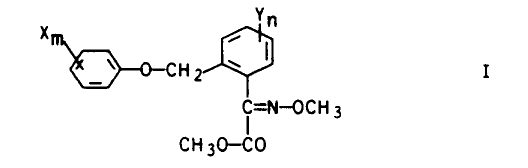 nicht störend, da sie unter den Reaktionsbedingungen des anschließenden Verfahrens gespalten und mit umgesetzt werden. Gewünschtenfalls können die Ketocarbonsäureester-dimethylketale VIIb aber auch unter sauren Bedingungen, beispielsweise durch Einleiten von Chlorwasserstoff in Gegenwart eines inerten Lösungsmittels, in die α-Ketocarbonsäureester VIIa überführt werden.

Des Weiteren können bei dem erfindungsgemäßen Verfahren die entsprechenden α-Ketocarbonsäureamide VIIc der Zwischenprodukte VIIa gebildet werden. Sind die α-Ketocarbonsäureamide VIIc unerwünscht, so unterwirft man zweckmäßigerweise das Rohprodukt-Gemisch erneut dem Verfahren, und zwar gegebenenfalls mehrmals.

Die Methanolyse der α-Ketocarbonsäureamide VIIc kann aber auch in einem getrennten Verfahrensschritt erfolgen, beispielsweise durch Behandlung der Verbindungen VIIc mit einer Säure, gewünschtenfalls in Gegenwart eines Verdünnungsmittels, z.B. einem Kohlenwasserstoff wie Toluol, einem Halogenkohlenwasserstoff wie Dichlormethan, Trichlormethan und Tetrachlorkohlenstoff oder einem Ether wie Diethylether, Diethylenglykol und Dioxan.

Als Säuren kommen beispielsweise Mineralsäuren wie Salzsäure, Schwefelsäure und Phosphorsäure, Carbonsäuren wie Essigsäure und Trifluoressigsäure oder Sulfonsäuren wie p-Toluolsulfonsäure in Betracht. Bevorzugte Säuren sind Schwefelsäure, insbesondere als konzentrierte wäßrige Lösung und Salzsäure, die besonders bevorzugt gasförmig eingeleitet wird.

Das erfindungsgemäße Verfahren kann sowohl kontinuierlich als auch diskontinuierlich durchgeführt werden. Bei der kontinuierlichen Arbeitsweise leitet man die Reaktionspartner beispielsweise durch einen Rohrreaktor oder über Rührkesselkaskaden.

Nach dem erfindungsgemäßen Verfahren erhält man die α-Ketocarbonsäureester VIIa in hohen Ausbeuten mit sehr guter Reinheit.

Die beschriebene Herstellungsmethode läßt sich mit Erfolg zur Synthese aller definitionsgemäßen α-Ketocarbonsäureesterester VIIa anwenden, vor allem auf solche Verbindungen, in denen die Substituenten X und Y jeweils ausgewählt sind aus einer Gruppe, bestehend aus
- Halogen wie Fluor, Chlor, Brom und Iod, insbesondere Fluor und Chlor;
- verzweigtes oder unverzweigtes C₁-C₄-Alkyl wie Methyl, Ethyl, n-Propyl, Isopropyl, Isobutyl, sec.-Butyl und n-Butyl, insbesondere Methyl und Ethyl;
- verzweigtes oder unverzweigtes C₁-C₄-Alkoxy wie Methoxy, Ethoxy, 1-Methylethoxy, n-Propoxy, Isopropoxy, n-Butoxy, Isobutoxy und sec.-Butoxy;
- Trifluormethyl;
   - m: bedeutet 0, 1, 2, 3 oder 4, insbesondere 0, 1 oder 2;
   - n: bedeutet 0, 1, 2 oder 3, insbesondere 0 oder 1.

O-Phenoxymethylphenylglyoxylsäureester der allgemeinen Formel VIIa' 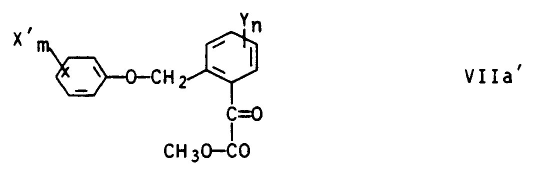 wobei die Variablen die folgende Bedeutung haben:
- X', Y: Substituenten, ausgewählt aus einer Gruppe bestehend aus Halogen, C₁-C₄-Alkyl, C₁-C₄-Alkoxy oder Trifluormethyl;
- m: eine ganze Zahl von 0 bis 4;
- n: eine ganze Zahl von 0 bis 3,
mit der Maßgabe, daß n nur dann 0 sein kann, wenn X' 2-Chlor, 2-Fluor, 2-Methyl, 4-Methyl, 4-tert.-Butyl, 2-Methoxy oder 2-Trifluormethyl und m 0 oder 1, oder X' 2,4-Dichlor oder 4-Chlor-2-methyl und m 2 bedeuten,
sind neu.

Ebenfalls neu sind die α-Ketocarbonsäureamide der allgemeinen Formel VIIc 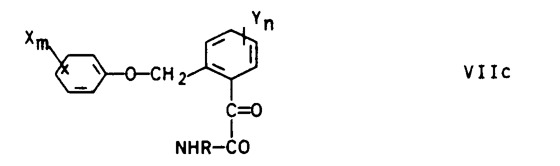 wobei die Variablen die folgende Bedeutung haben:
- X, Y: Substituenten, ausgewählt aus einer Gruppe bestehend aus Halogen, C₁-C₄-Alkyl, C₁-C₄-Alkoxy oder Trifluormethyl;
- m: eine ganze Zahl von 0 bis 4;
- n: eine ganze Zahl von 0 bis 3 und
- R: Wasserstoff oder Acyl.

Besonders gut geeignete α-Ketocarbonsäuremethylester VIIa sowie bevorzugte neue α-Ketocarbonsäureamide VIIc sind Tabelle 1 zu entnehmen.

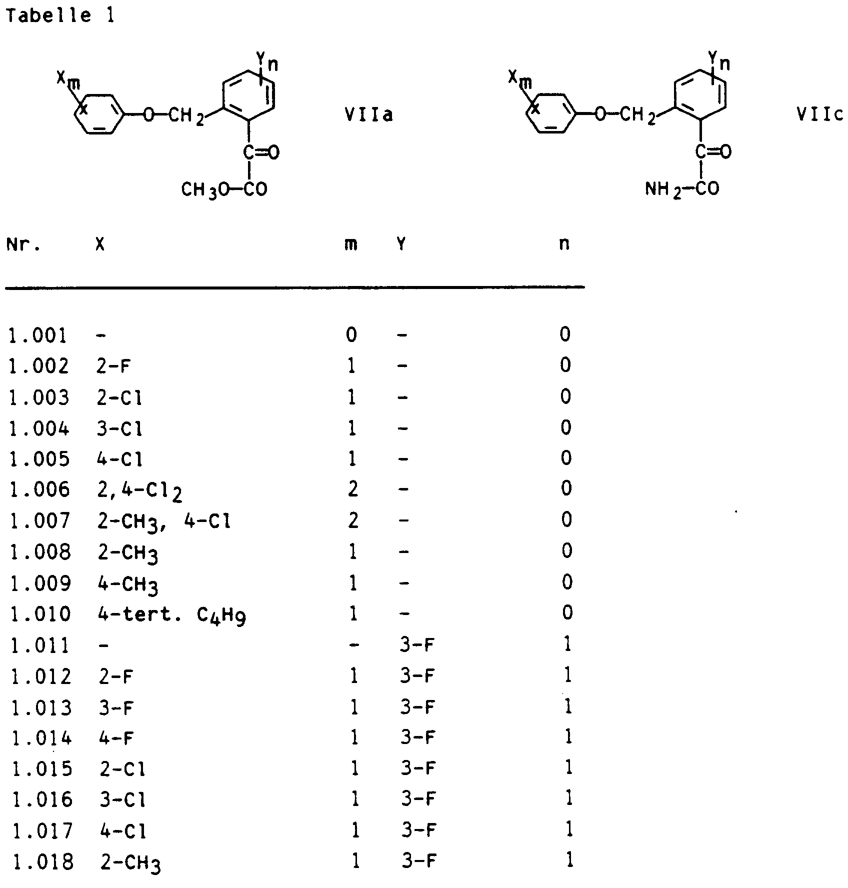

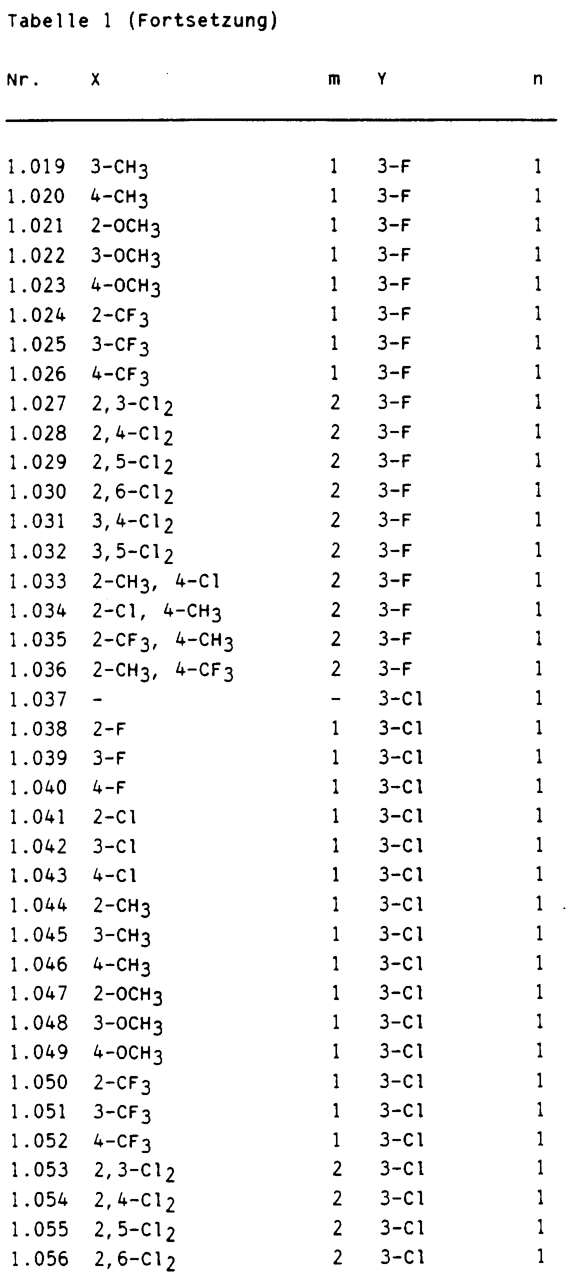

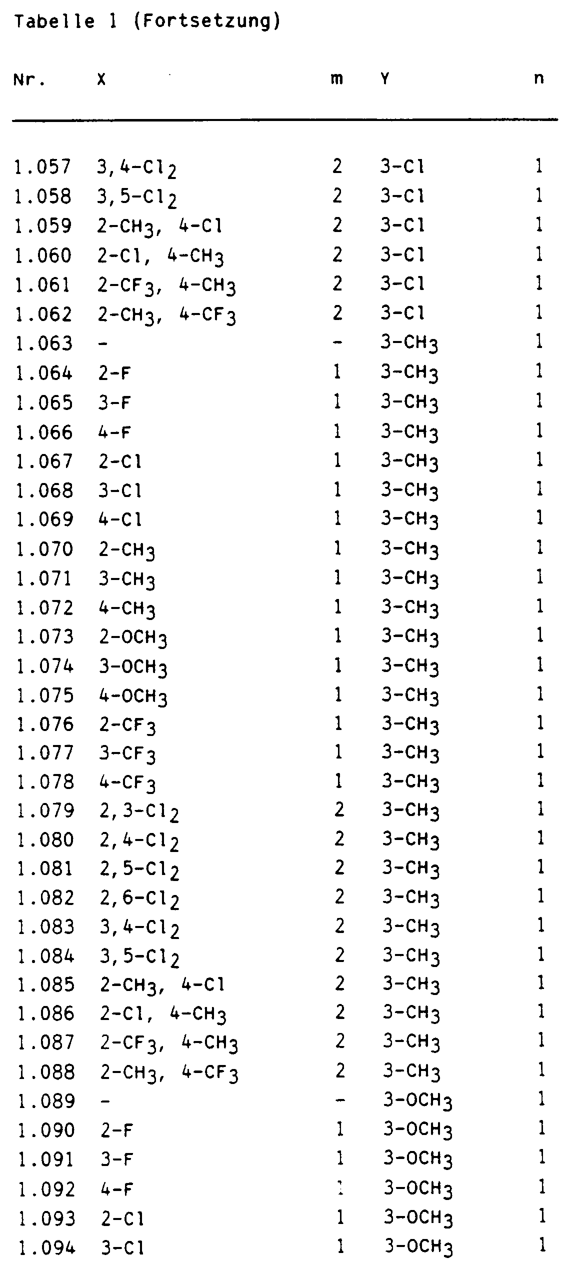

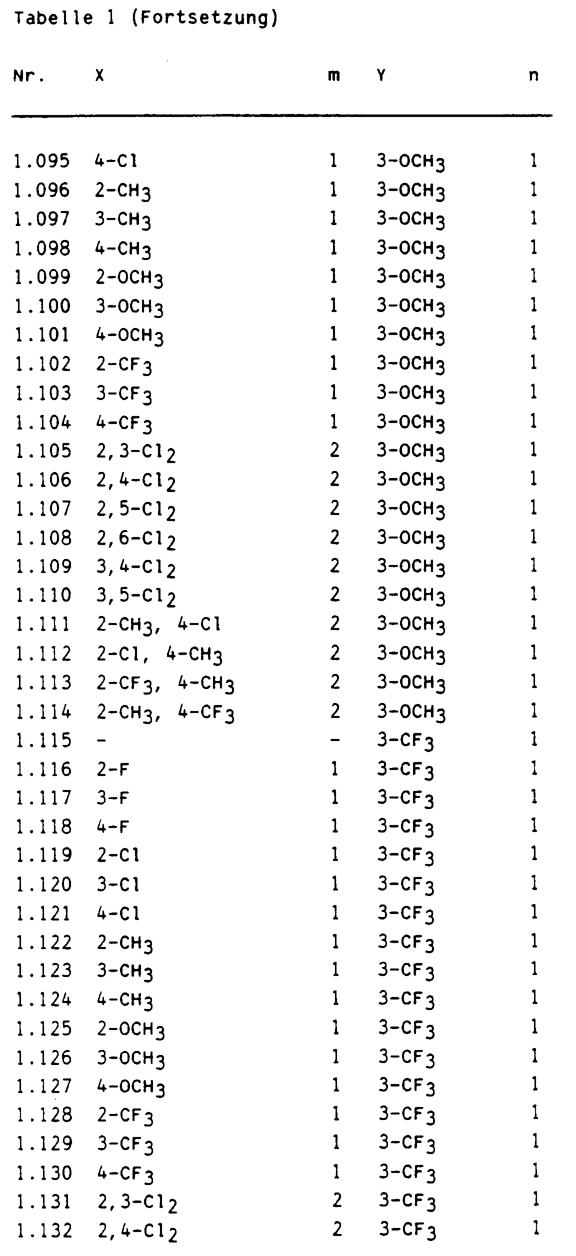

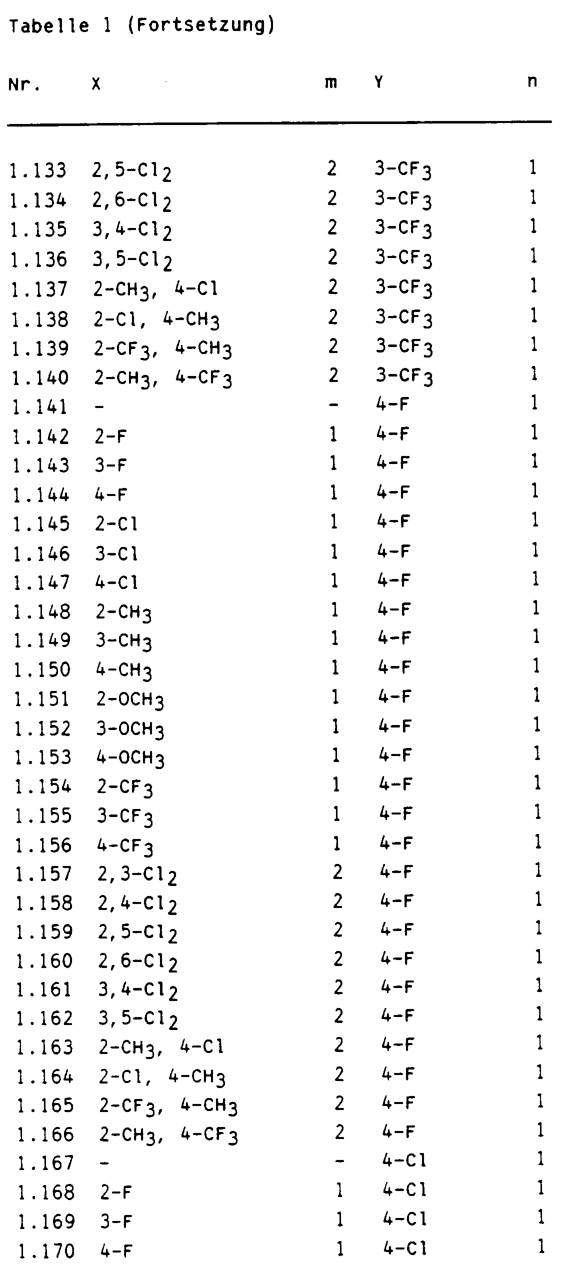

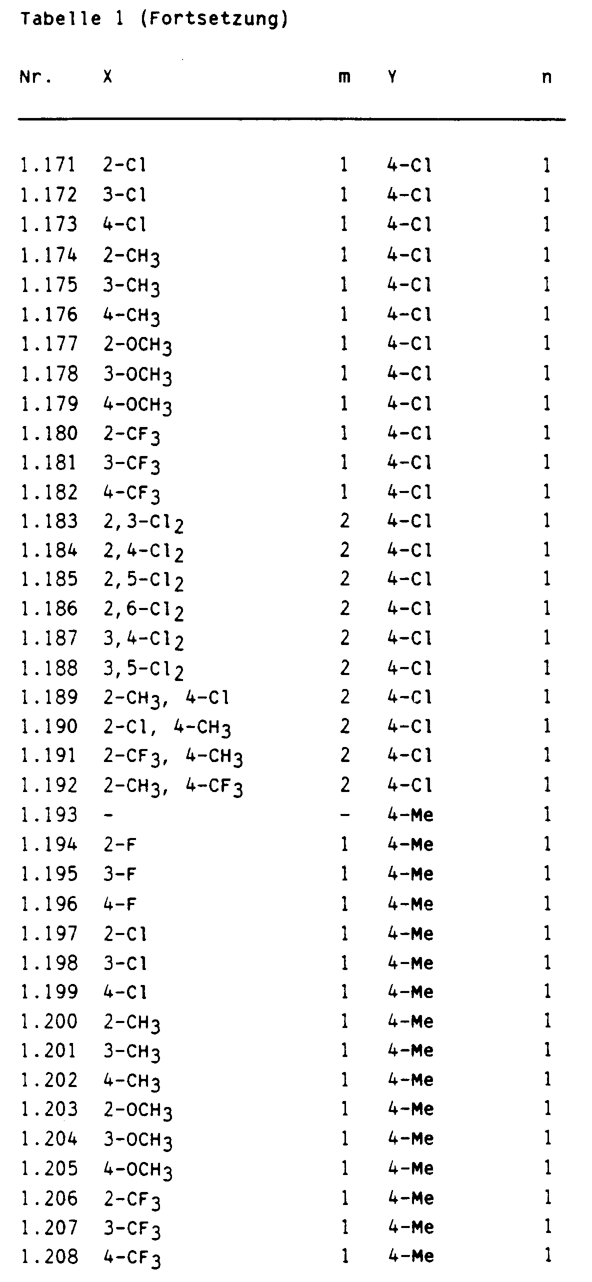

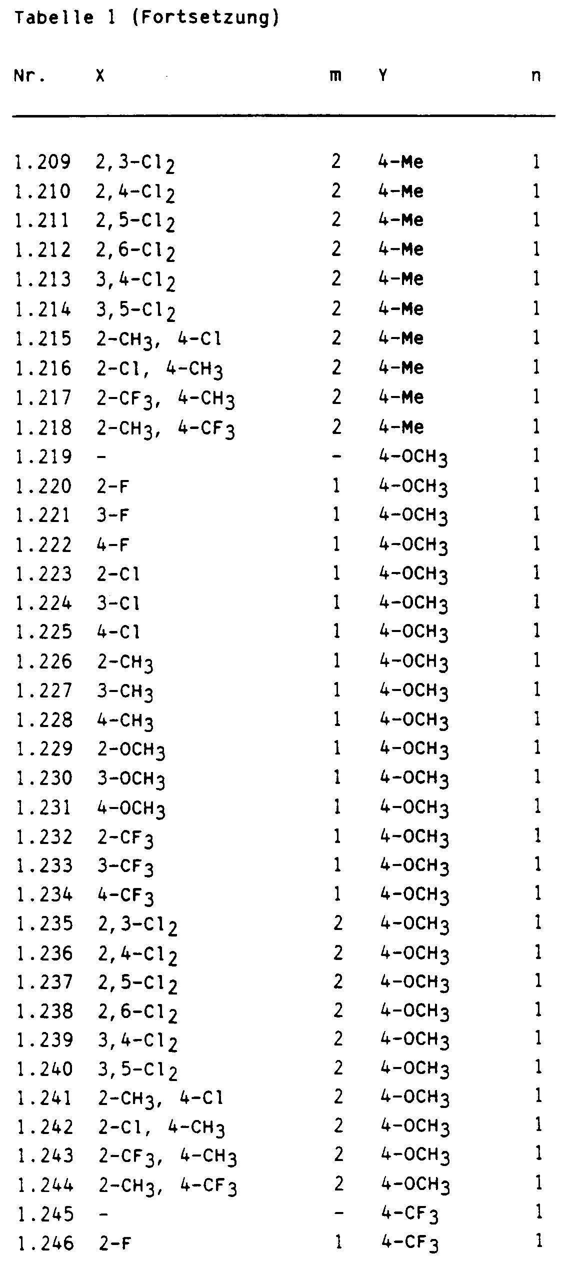

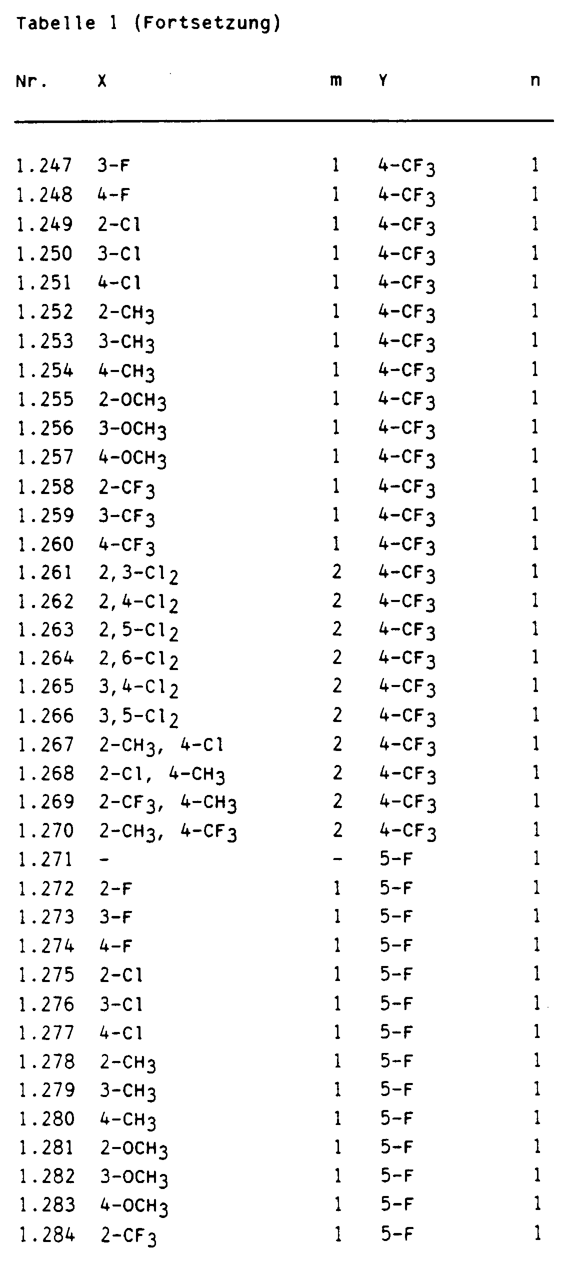

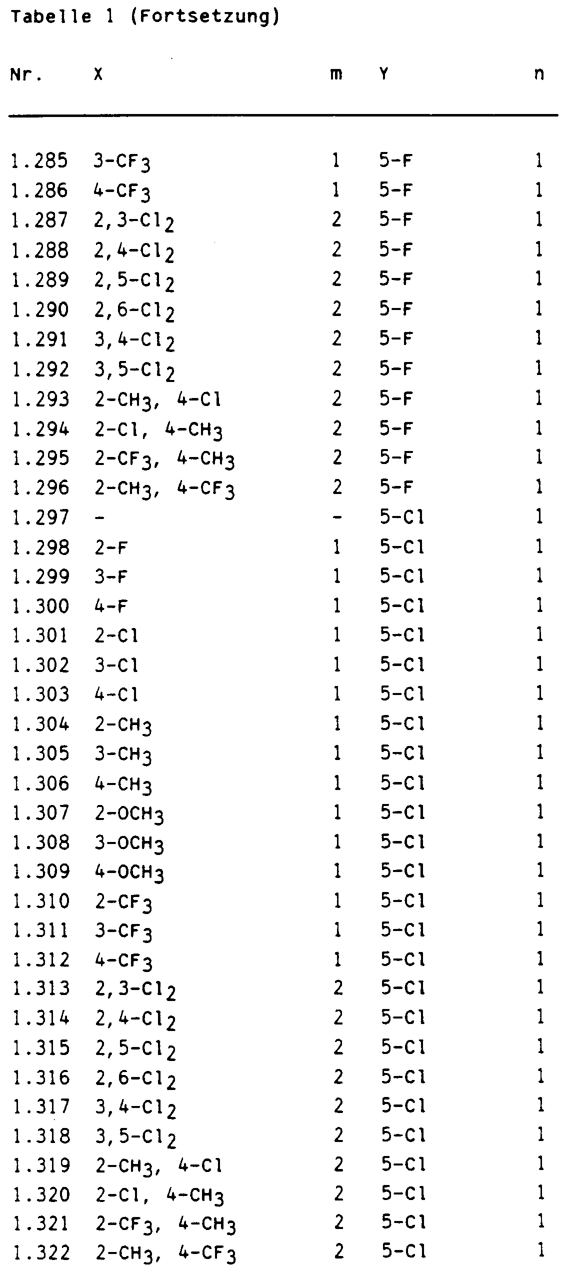

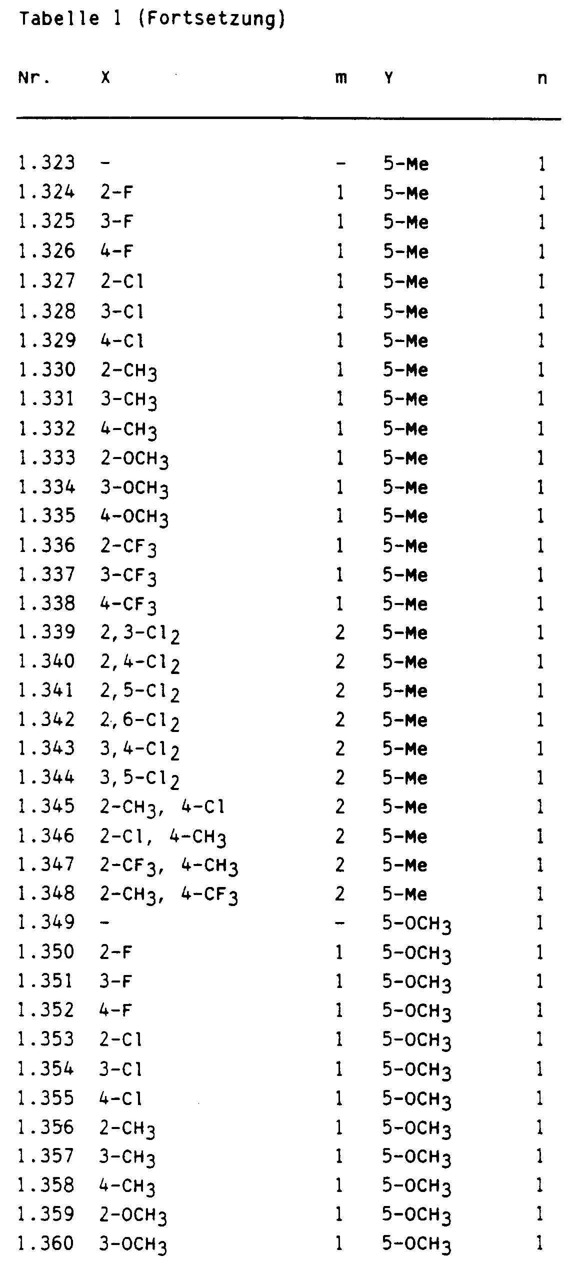

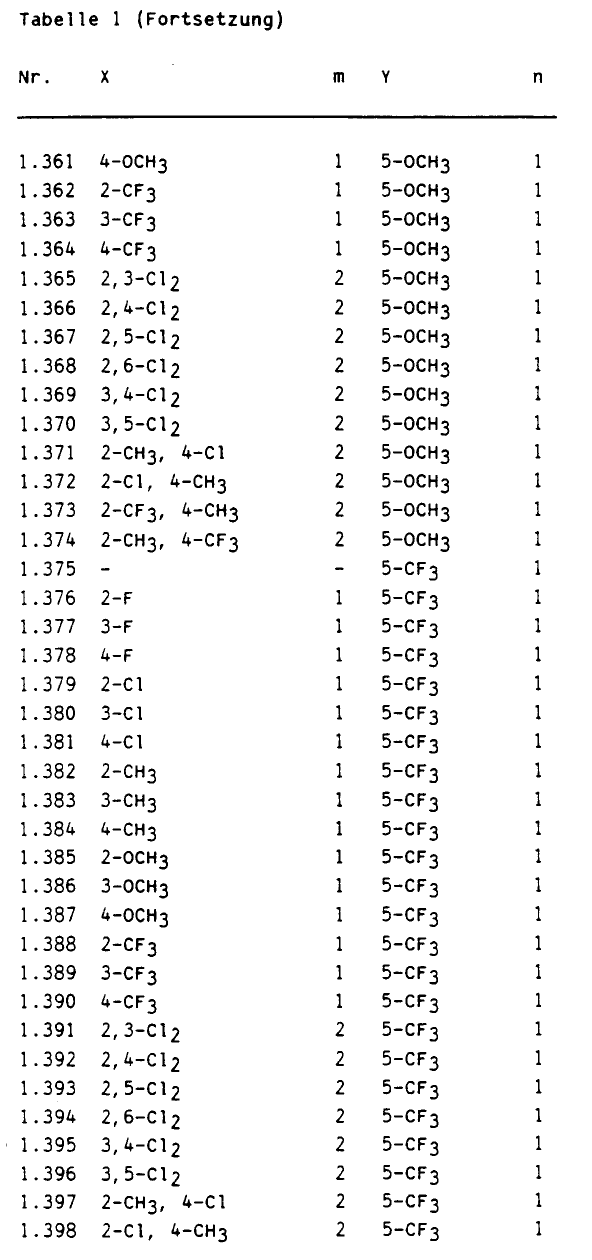

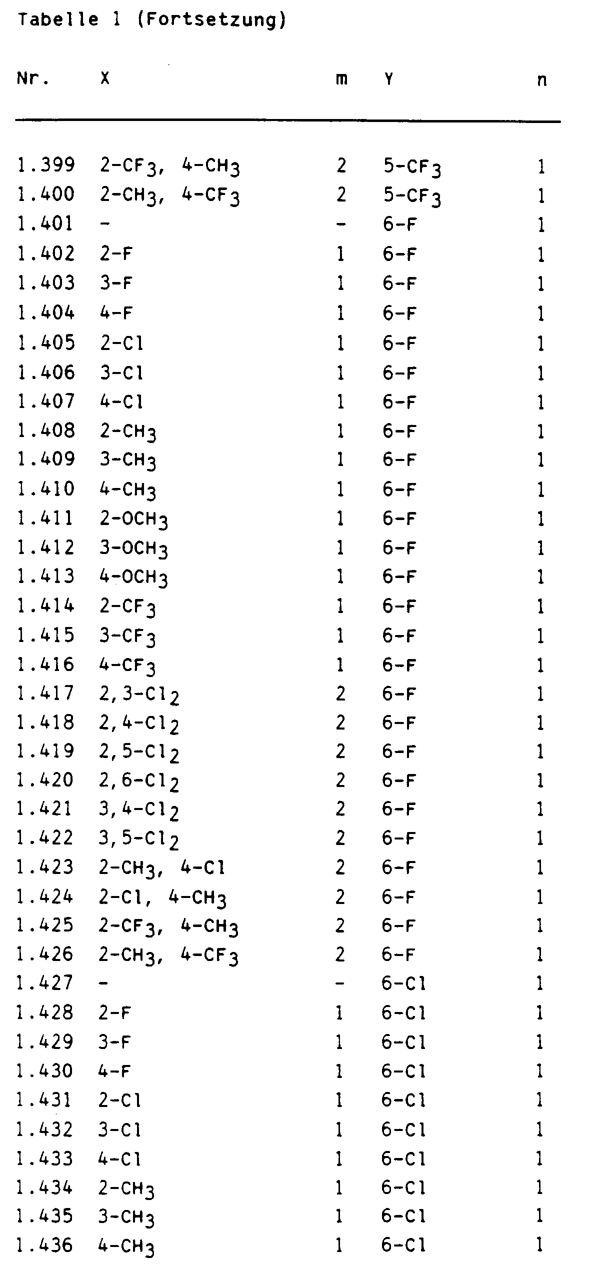

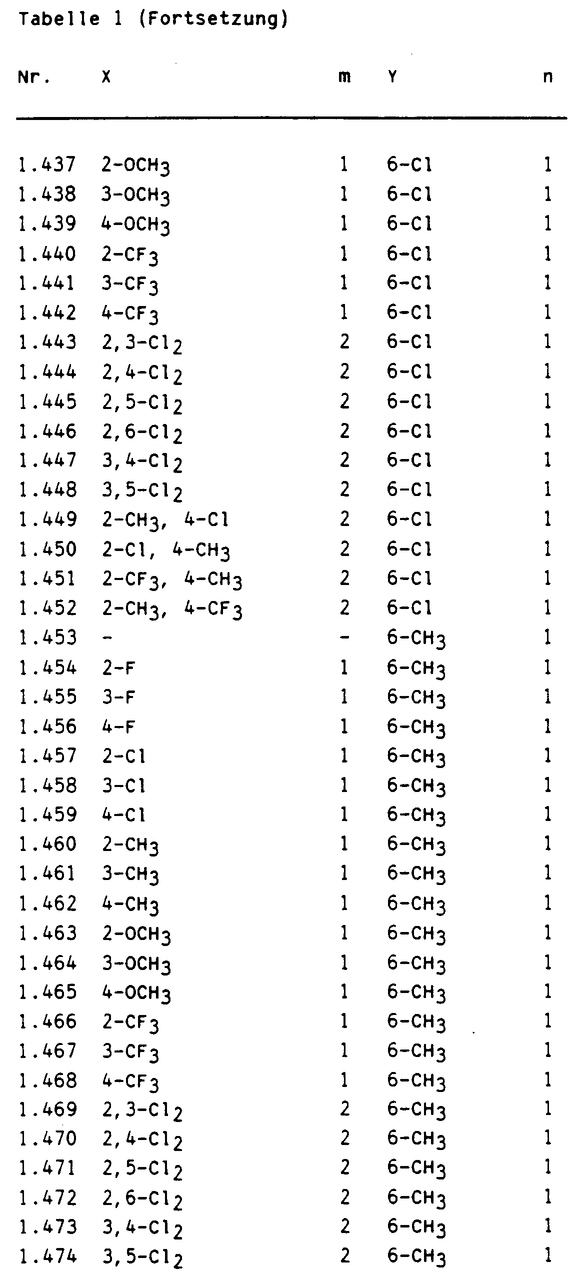

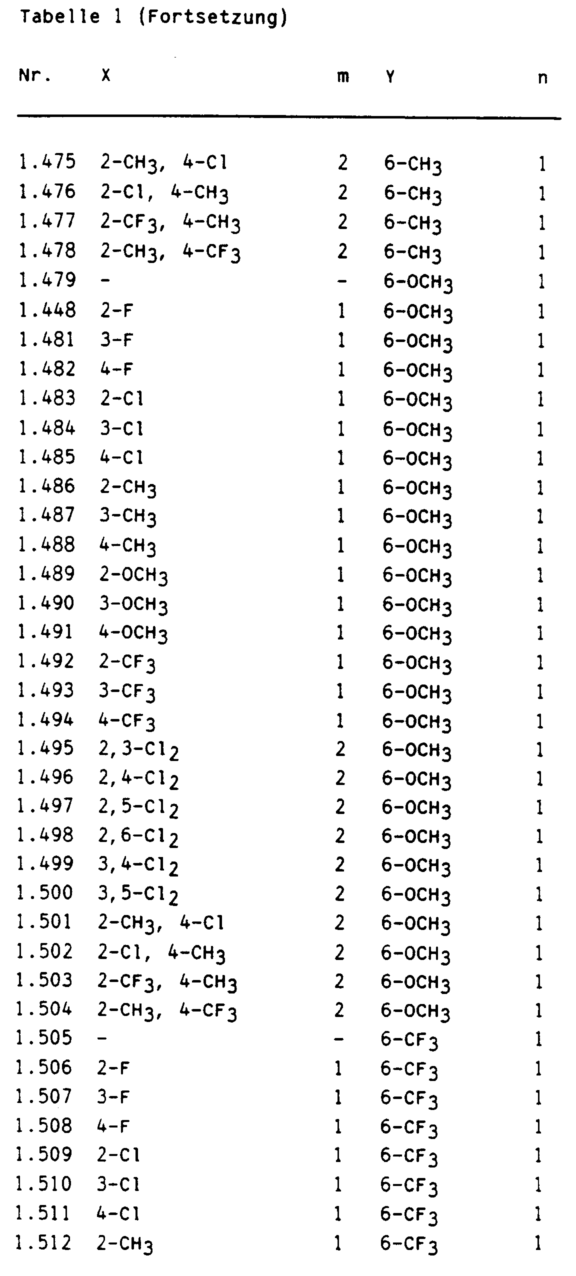

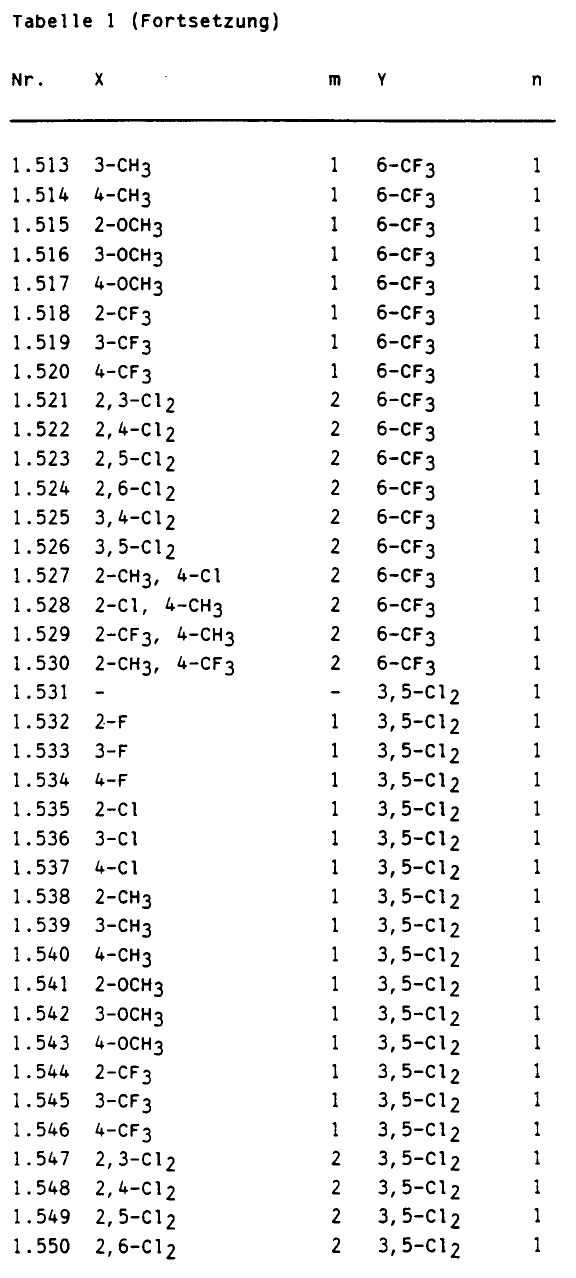

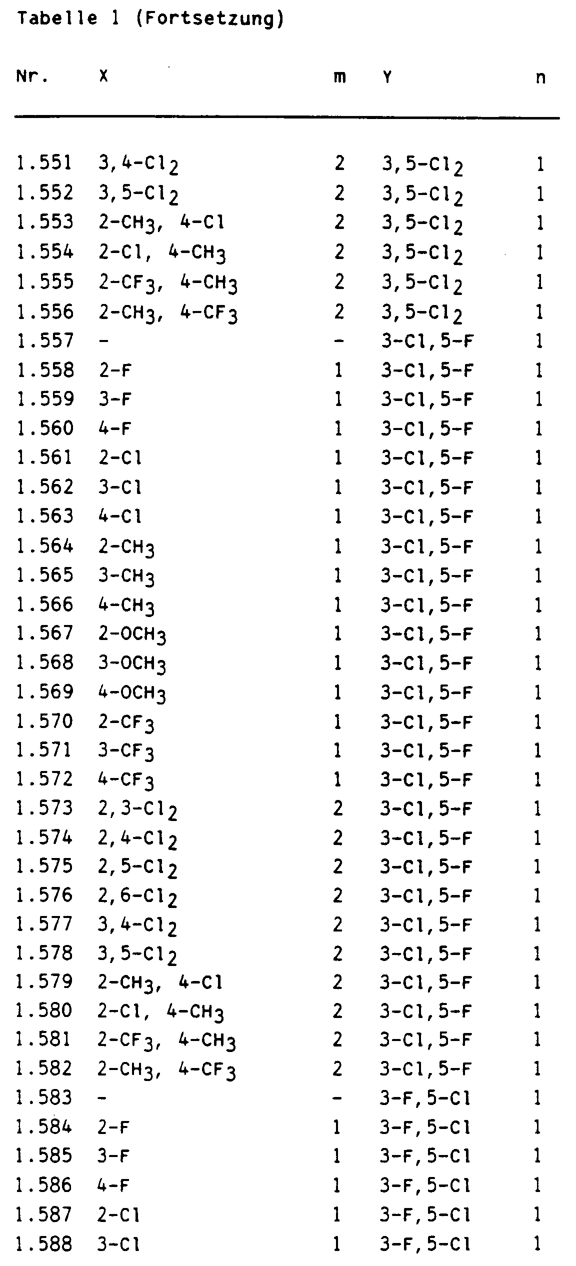

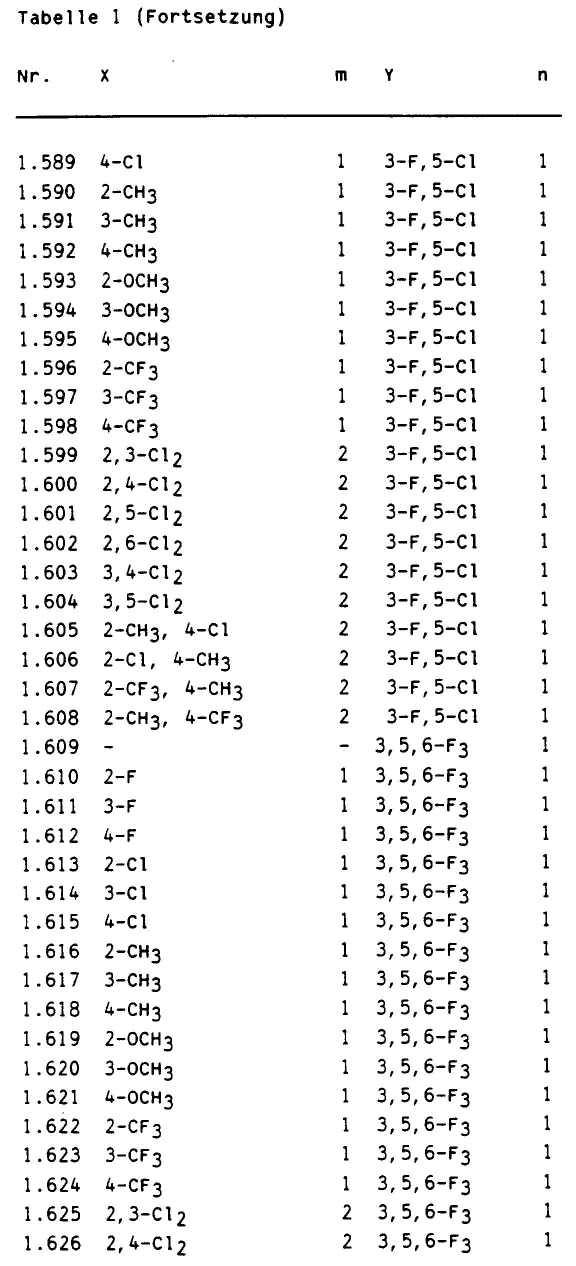

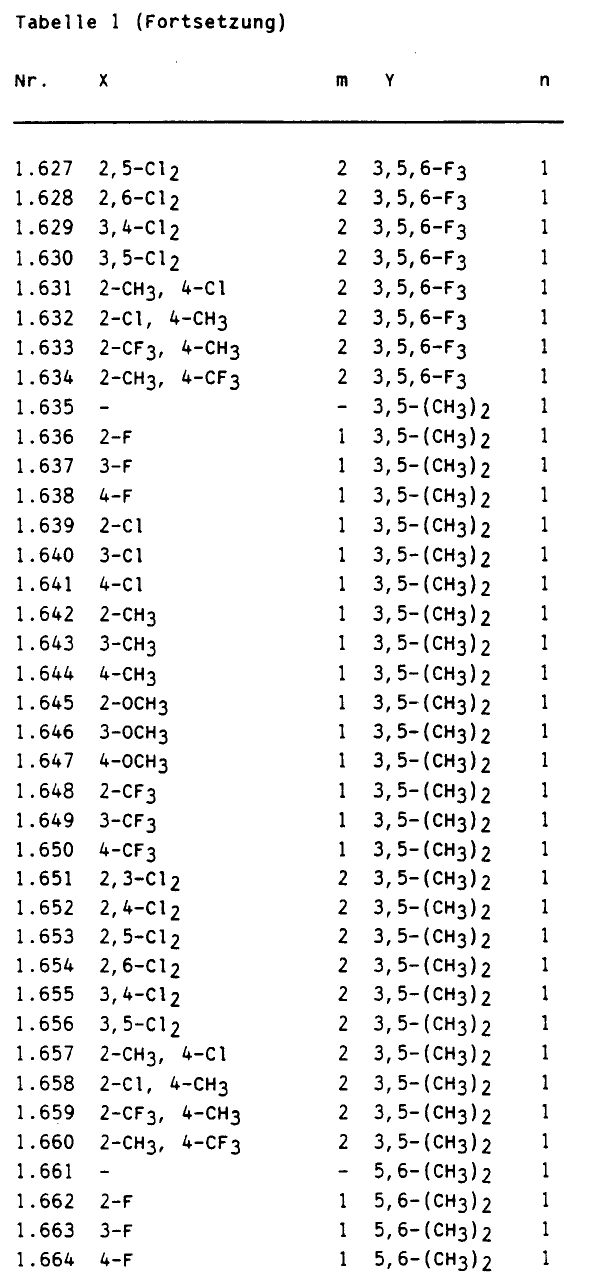

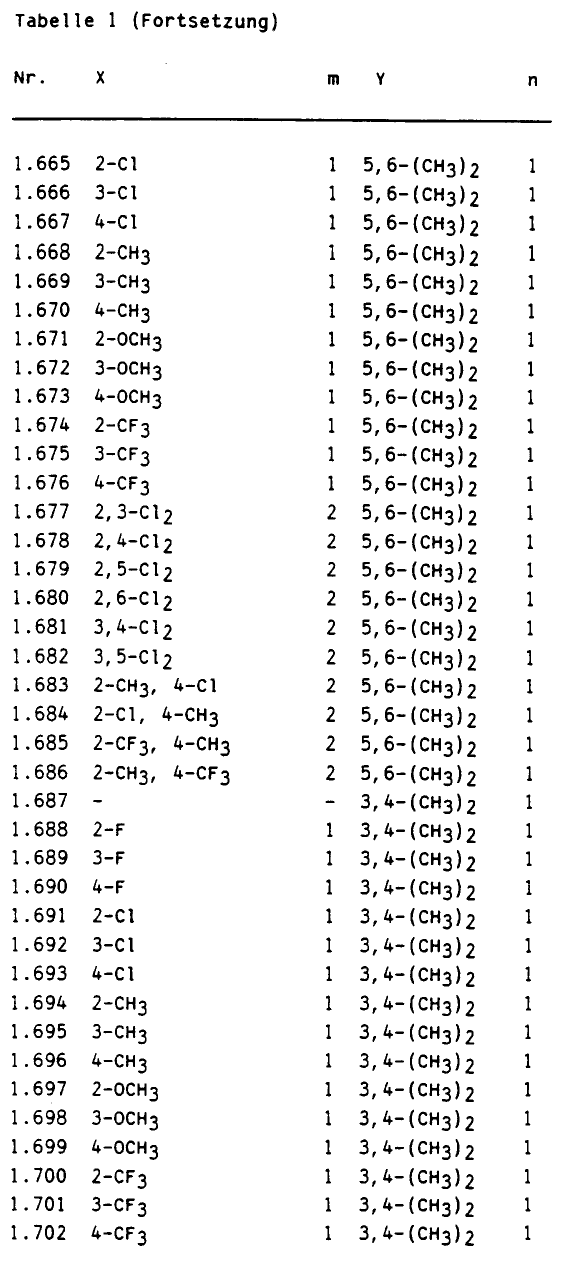

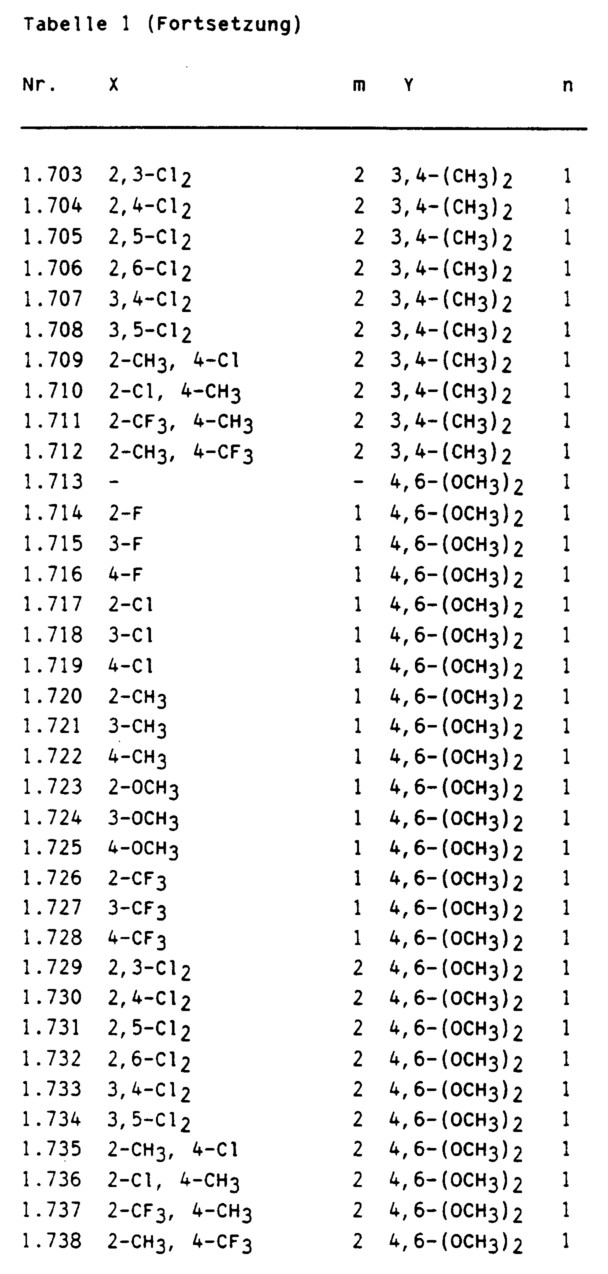

Die α-Ketocarbonsäureester VIIa sind wertvolle Zwischenprodukte insbesondere für die Synthese von E-Oximethern von Phenoxymethylphenylglyoxylsäureestern I, die im Pflanzenschutz vorzugsweise als Fungizide Verwendung finden (vgl. EP-A 253 213 und der EP-A 254 426).

Die Endprodukte I lassen sich herstellen, indem man die α-Ketocarbonsäureester VIIa, gegebenenfalls als Rohproduktgemisch zusammen mit den Ketocarbonsäureester-dimethylketalen VIIb, mit O-Methylhydroxylamin oder einem seiner Säureadditionssalze umsetzt, wobei das hierbei erhaltenen E/Z-Isomerengemisch der Oximether zur weiteren Umlagerung der Z- in die E-Isomeren gleichzeitig oder anschließend mit einer Säure behandelt wird:

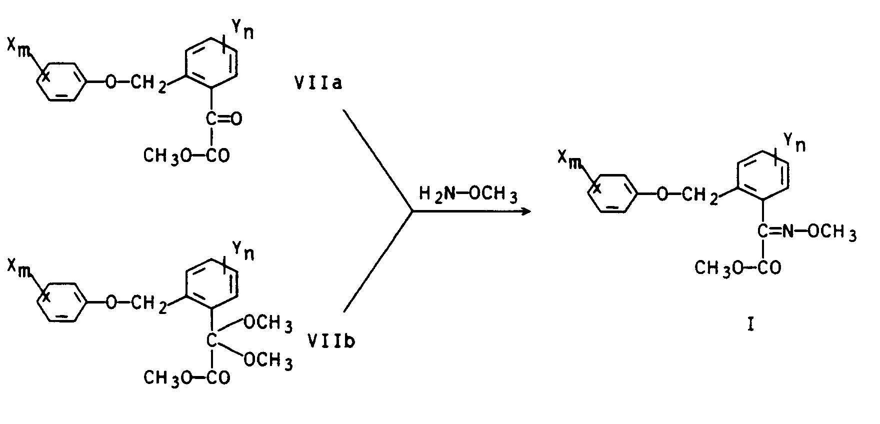

### Beispiel 6 (erfindungsgemäß)

### 2-[(2'-Methyl)-phenoxymethyl]-phenyl-glyoxylsäuremethylester

### 6.1 ohne Verdünnungsmittel

Zu einer Lösung aus 10 g (40 mmol) 2-[(2'-Methyl)phenoxymethyl]-benzoylcyanid in 150 ml Methanol wurden 4,1 g (40 mmol) Essigsäureanhydrid gegeben. Dann leitete man bei (-5) bis (-10)°C 65 g (1,78 mol) Chlorwasserstoff-Gas ein, ließ die Mischung auf 20°C erwärmen und rührte noch etwa 15 Stunden bei dieser Temperatur. Anschließend wurde gebildetes Ammoniumchlorid abgetrennt und mit 100 ml Methylenchlorid gewaschen, wonach man die vereinigten organischen Phasen einengte. Ausbeute: 11,3 g eines gelbbraunen Feststoffes, dessen gaschromatographische Analyse folgende Zusammensetzung ergab:
- 19,3 mol-% 2-[(2'-Methyl)phenoxymethyl]-benzoesäuremethylester (Nebenprodukt),
- 12,9 mol-% 2-[(2'-Methyl)phenoxymethyl]-phenylglyoxylsäuremethylester (Produkt der Formel VIIa),
- 11,8 mol-% 2-[(2'-Methyl)phenoxymethyl]-phenylglyoxylsäuremethylesterdimethylketal (Produkt der Formel VIIb),
- 60,9 mol-% 2-[(2'-Methyl)phenoxymethyl]-phenylglyoxylsäure-amid (Produkt der Formel VIIc).

### 6.2 mit Verdünnungsmittel (Toluol)

Zu einer Suspension aus 50 g (0,2 mol) 2-[(2'-Methyl)phenoxymethyl]-benzoylcyanid in 150 ml Toluol wurden 30 ml Methanol und 20,4 g (0,2 mol) Essigsäureanhydrid gegeben. Dann leitete man bei 25 bis 30°C 22 g (0,6 mol) Chlorwasserstoff-Gas ein und rührte noch etwa 15 Stunden. Anschließend wurde gebildetes Ammoniumchlorid abgetrennt und mit 100 ml Methylenchlorid gewaschen, wonach man die vereinigten organischen Phasen einengte. Ausbeute: 50 g eines gelbbraunen Feststoffes, dessen gaschromatographische Analyse folgende Zusammensetzung ergab:
- 7,8 mol-% 2-[(2'-Methyl)phenoxymethyl]-benzoesäuremethylester (Nebenprodukt),
- 34,1 mol-% 2-[(2'-Methyl)phenoxymethyl]-phenylglyoxylsäuremethylester (Produkt der Formel VIIa),
- 54,1 mol-% 2-[(2'-Methyl)phenoxymethyl]-phenylglyoxylsäure-amid (Produkt der Formel VIIc).

¹H-NMR des 2-[(2'-Methyl)phenoxymethyl]-phenylglyoxylsäuremethylesters (in CDCl₃; TMS als interner Standard): 2,28 ppm (s,3H); 3,77 ppm (s,3H); 5,38 ppm (s,2H); 6,83-7,79 ppm (m,8H);

¹H-NMR des 2-[(2'-Methyl)phenoxymethyl]-phenylglyoxylsäure-amides (in CDCl₃; TMS als interner Standard): 2,23 ppm (s,3H); 5,31 ppm (s,2H); 5,81 ppm (br,2H); 6,87-7,99 ppm (m,8H).

### Beispiel 7 (erfindungsgemäß)

### Überführung von 2-[(2'-Methyl)phenoxymethyl]-phenylglyoxylsäure-amid in den 2-[(2'-Methyl)phenoxymethyl]-phenylglyoxylsäuremethylester

Zu einer Lösung von 39,4 g eines Gemisches, bestehend aus 38,9 mol-% 2-[(2'-Methyl)phenoxymethyl]-phenylglyoxylsäure-amid und 57,6 mol-% 2-[(2'-Methyl)phenoxymethyl]-phenylglyoxylsäuremethylester (nach HPLC-Analyse), in 200 ml Methanol wurden 10 ml konzentrierte wässrige Schwefelsäure gegeben. Die Mischung wurde 2,5 Stunden auf Rückflußtemperatur erhitzt und dann noch etwa 15 Stunden bei 20-25°C gerührt. Die HPLC-Analyse einer Probe zeigte, daß die gelöste Substanz bereits zu 81,6 mol-% aus 2-[(2'-Methyl)phenoxymethyl]-phenylglyoxylsäuremethylester und nur mehr zu 1,4 mol-% aus 2-[(2'-Methyl)phenoxymethyl]-phenylglyoxylsäure-amid bestand. Nach der üblichen Aufarbeitung der Mischung konnte das Rohprodukt direkt zur Synthese des entsprechenden Endproduktes der Formel I verwendet werden.

### Beispiel 8 (erfindungsgemäß)

### Spaltung von 2-[(2'-Methyl)phenoxymethyl]-phenylglyoxylsäuremethylesterdimethylketal in 2-[(2'-Methyl)phenoxymethyl]-phenylglyoxylsäuremethylester

Zu einer Lösung von 130,2 g eines Gemisches, bestehend aus 55,5 mol-% 2-[(2'-Methyl)phenoxymethyl]-phenylglyoxylsäuremethylester 32,3 mol-% 2-[(2'-Methyl)phenoxymethyl]-phenylglyoxylsäuremethylester-dimethylketal und 12,2 mol-% 2-[(2'-Methyl)phenoxymethyl]-benzoesäuremethylester (nach gaschromatographischer Analyse), in 300 ml Methylenchlorid wurden 100 ml konzentrierte wässrige Salzsäure gegeben. Die Mischung wurde 15 Stunden bei 20-25°C gerührt, dann mit 100 ml Wasser, 100 ml konzentrierter wässriger Natriumcarbonat-Lösung und nochmals mit 100 ml Wasser gewaschen und schließlich getrocknet und eingeengt. Ausbeute: 117,9 g eines gelben Öles, das (nach gaschromatographischer Analyse) zu 83,9 mol-% aus 2-[(2'-Methyl)phenoxymethyl]-phenylglyoxylsäuremethylester und unverändert zu 12,3 mol-% aus 2-[(2'-Methyl)phenoxymethyl]-benzoesäuremethylester bestand.

### Beispiel 9 (Vergleichsbeispiele nach dem Stand der Technik)

### 9.1) Umsetzung von 2-[2-Methylphenoxymethyl]-benzoylcyanid unter NaBr-Katalyse (vgl. US-A 4 234 739)

- Hauptreaktion:: Überführung in Benzoesäuremethylester

### 9.1.1 in Lösung:

Zu einer Mischung aus 19,6 ml 85 gew.-%iger Schwefelsäure und 1,09 g Natriumbromid wurde bei 20-25°C eine Lösung von 20,5 g (81 mmol) 2-[2-Methylphenoxymethyl]-benzoylcyanid in 80 ml Methylenchlorid getropft. Danach versetzte man das Gemisch bei 40°C mit 38,9 ml Methanol und rührte noch 3 Stunden bei dieser Temperatur. Die HPLC-Analyse einer entnommenen Probe zeigte quantitative Bildung von 2-[2-Methylphenoxymethyl]-benzoesäuremethylester (unerwünschtes Konkurrenzprodukt). Nach Zugabe von 100 ml Wasser wurde die erhaltene Mischung zweimal mit je 50 ml Methylenchlorid extrahiert, wonach man die vereinigten organischen Phasen wie üblich auf das Produkt hin aufarbeitete. Ausbeute: 95 % des Konkurrenzproduktes.

### 9.1.2 ohne Lösungsmittel:

Zu einer Mischung aus 20 ml 85 gew.-%iger Schwefelsäure und 1,0 g Natriumbromid wurden bei etwa 20°C 18,9 g (78 mmol) 2-[(2'-Methyl)phenoxymethyl]-benzoylcyanid gegeben. Anschließend tropfte man 100 ml Methanol zu, wobei die Reaktionstemperatur auf 80°C anstieg und Cyanwasserstoff gebildet wurde. Das Reaktionsgemisch wurde noch 3 Stunden bei 80°C gerührt, und nach Abkühlung auf ca. 25°C mit 100 ml Toluol extrahiert. Die gaschromatographische Analyse zeigte auch bei dieser Synthesevariante quantitative Bildung von 2-[2-Methylphenoxymethyl]-benzoesäuremethylester (unerwünschtes Konkurrenzprodukt).

Fp.: 33-37°C; ¹H-NMR (in CDCl₃, TMS als interner Standard): 2,33 ppm (s,3H); 3,87 ppm (s,3H); 5,48 ppm (s,2H); 6,83-8,01 ppm (m,8H).

### 9.2) Umsetzung von 2-[(2'-Methyl)phenoxymethyl]-benzoylcyanid nach der Methode von Ritter (vgl. EP-A 034 240)

- Hauptreaktionen:: Cyanidabspaltung und Alkylierung des Phenoxy-Restes

Zu einem Gemisch aus 4,2 g Essigsäure, 3 g konzentrierter wässriger Schwefelsäure wurde bei etwa 20°C eine Lösung von 5 g (20 mmol) 2-[(2-Methyl)phenoxymethyl]-benzoylcyanid in 80 ml Methyl-tert.-butylether gegeben. Nach 2 Stunden rühren versetzte man die Mischung mit 10 ml konzentrierter wässriger Schwefelsäure. Das Reaktionsgemisch wurde noch 2 Stunden auf Rückflußtemperatur erhitzt und dann mit 100 ml Wasser versetzt. Die organische Phase wurde abgetrennt, getrocknet und eingeengt. Ausbeute: 5,9 g eines Rohproduktes, dessen gaschromatographische und massenspektroskopische Analyse folgende Zusammensetzung ergab:
- 15 mol-% 2-[(2'-Methyl)phenoxymethyl]-benzoesäuremethylester (unerwünschtes Konkurrenzprodukt),
- 17 mol-% 2-[(2'-Methyl-4-tert.-butyl)phenoxymethyl]-benzoesäuremethylester (unerwünschtes Konkurrenzprodukt),
- 13 mol-% 2-[(2'-Methyl)phenoxymethyl]-phenylglyoxylsäure-N-(tert.-butyl)amid,
- 23 mol-% 2-[(2'-Methyl-4-tert.-butyl)phenoxymethyl]-phenylglyoxylsäure-N-(tert.-butyl)amid (unerwünschtes Nebenprodukt).

### 9.3) Umsetzung von 2-[(2'-Methyl)phenoxymethyl]-benzoylcyanid mit Schwefel säure und Essigsäureanhydrid (vgl. EP-A 035 707)

- Hauptreaktion:: Cyanidabspaltung und Sulfonylierung des Phenoxy-Restes

Zu einer Mischung aus 80 ml Essigsäureanhydrid und 50 g (0,199 mol) 2-[(2-Methyl)phenoxymethyl]-benzoylcyanid wurden bei 20°C langsam 39 g konzentrierte wässrige Schwefelsäure gegeben. Das Gemisch, das sich hierbei auf 90°C erwärmt hatte, wurde nach Abkühlung auf 25°C mit 100 ml Wasser und anschließend bis pH = 3 mit konzentrierter wässriger Natronlauge versetzt. Anschließend wurde der gebildete braune Niederschlag abgetrennt und getrocknet. Ausbeute: 83,8 % 2-[(2-Methyl-4-sulfo)phenoxymethyl]-benzoesäure (unerwünschtes Produkt); Fp.: 242-244°C.

¹H-NMR (in d6-DMSO; TMS als interner Standard): 2,25 ppm (s,3H), 5,50 ppm (s,2H); 6,93-7,98 ppm (m,7H).

Die Endprodukte I lassen sich herstellen, indem man die α-Ketocarbonsäureester VII mit O-Methylhydroxylamin oder einem seiner Säureadditionssalze umsetzt, wobei das hierbei erhaltenen E/Z-Isomerengemisch zur weiteren Umlagerung der Z- in die E-Isomeren gleichzeitig oder anschließend mit einer Säure behandelt wird:

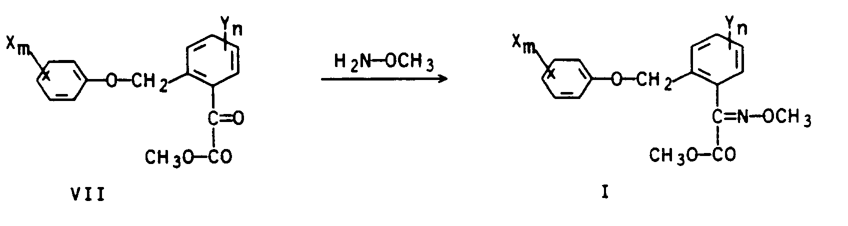

### Beispiele

### Beispiel 10 (Vorstufen)

### Vorstufe α

### 2-[(2'-Methyl)-phenoxymethyl]-benzoesäure

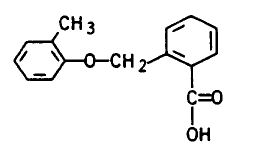

Eine Mischung aus 160 g (1,48 mol) o-Kresol, 106 g 85 gew.-%ige wässrige Kaliumhydroxid-Lösung und 1,5 l Xylol wurden unter laufender Entfernung des Wassers auf Rückflußtemperatur erhitzt. Dann kühlte man die Mischung auf 100°C und versetzte sie bei dieser Temperatur mit 195 g (1,45 mol) Phthalid und 57 ml Dimethylformamid. Das erhaltene Gemisch wurde noch 15 Stunden auf 100°C erhitzt, anschließend auf 20-25°C abgekühlt, zweimal mit je 2 l Wasser extrahiert und dann mit 140 ml 38 gew.-%iger wässriger Salzsäure versetzt. Die gebildeten Kristalle wurden abgetrennt, mit 500 ml Wasser gewaschen und getrocknet. Zur Reinigung löste man das Produkt in 550 ml warmem Aceton und fällte es durch Zugabe von 3 l Wasser wieder aus. Ausbeute: 85 %; Fp.: 154°C;

¹H-NMR (in CDCl₃, TMS als interner Standard): 2,39 ppm (s,3H); 5,38 ppm (s,2H); 6,9-8,2 ppm (m,8H).

### Vorstufe β

### 2-[(2'-Methyl)-phenoxymethyl]-benzoesäuremethylester

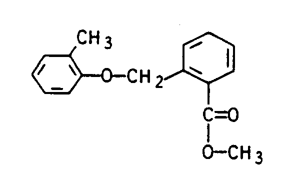

Zu einer Lösung aus 24,3 g (0,1 mol) 2-[(2'-Methyl)-phenoxymethyl]-benzoesäure in 100 ml Methanol wurden 12,5 g (0,1 mol) Thionylchlorid getropft. Nach 4,5 Stunden bei 20-25°C wurde das Lösungsmittel entfernt. Ausbeute: 89 %; Fp.: 51°C.

¹H-NMR (in CDCl₃, TMS als interner Standard): 2,4 ppm (s,3H); 3,98 ppm (s,3H); 5,58 ppm (s,2H); 6,95-8.05 ppm (m,8H).

### Beispiel 11 (erfindungsgemäß)

### 2-[(2'-Methyl)-phenoxymethyl]-phenyl-glyoxylsäuremethylester

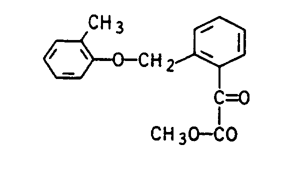

### Verfahrensschritt (a)

64 ml (0,9 mol) Dimethylsulfoxid wurden in einem trockenen Kolben vorgelegt und mit 8,6 g (0,12 mol) Natriummethylat versetzt. Das Reaktionsgemisch wurde auf 65°C erhitzt und nach 2 Stunden bei dieser Temperatur mit einer Lösung von 20,5 g (0,08 mol) 2-[(2'-Methyl)-phenoxymethyl]-benzoesäuremethylester in 64 ml Dimethylsulfoxid versetzt. Man rührte noch etwa 15 Stunden bei 20-25°C, entfernte dann das Lösungsmittel unter reduziertem Druck und goß den Rückstand auf Eiswasser. Nach Ansäuern der wässrigen Phase mit Eisessig (pH-Wert = 2) bildeten sich Kristalle, die abgetrennt, nacheinander mit gesättigter wässriger Natriumhydrogencarbonat-Lösung, Wasser und Diisopropylether gewaschen und getrocknet wurden. Ausbeute: 77 % 2-[(2'-Methyl)-phenoxymethyl]-ω-(methylsulfenyl)-acetophenon;

¹H-NMR (in CDCl₃, TMS als interner Standard): 2,3 ppm (s,3H); 2,8 ppm (s,3H); 4,4 ppm (q,2H); 5,4 ppm (s,2H); 6,9-8,0 ppm (m,8H).

### Verfahrensschritte (b) und (c)

### Variante 11.1

Zu einer Suspension aus 24,1 g (79,8 mmol) 2-[(2'-Methyl)-phenoxymethyl]-Ω-(methylsulfonyl)-acetophenon in 400 ml Aceton wurden 12,6 g (44 mmol) 3,3-Dimethyl-5,5-dibromhydantoin gegeben. Nach 30 Minuten Rühren bei 20-25°C entfernte man das Lösungsmittel unter reduziertem Druck und versetzte den Rückstand mit 500 ml Methanol und 40 ml konzentrierter wässriger Salzsäure. Anschließend rührte man die Mischung noch 20 Stunden bei 20-25°C, entfernte dann das Lösungsmittel bei reduziertem Druck und versetzte den Rückstand mit 500 ml eiskaltem Wasser. Die wässrige Phase wurde dreimal mit je 150 ml Methylenchlorid extrahiert, wonach die vereinigten Extrakte wie üblich auf das Produkt hin aufgearbeitet wurden. Man erhielt ein dickflüssiges Öl, das langsam auskristallisierte. Ausbeute: 85 %;

### Variante 11.2

Zu einer Lösung von 36 g (0,2 mol) 30 gew.-%iger Natriummethanolat-Lösung in 150 ml Methanol gab man bei Raumtemperatur 30,2 g (0,1 mol) 2-[(2'-Methyl-phenoxymethyl-Ω-(methylsulfonyl)-acetophenon. Hierzu tropfte man bei 20°C eine Lösung von 19,8 g (0,124 mol) Brom in 50 ml Methanol. Nach beendeter Zugabe rührte man 30 min bei 20°C und tropfte anschließend 20 ml konzentrierte wäßrige Salzsäure zu. Anschließend wurde das Gemisch 30 min auf Rückflußtemperatur (ca. 65°C) erhitzt. Nach dem Abkühlen wurde das Gemisch mit 500 ml Essigester versetzt und zweimal mit je 100 ml Wasser gewaschen. Die organische Phase wurde über Na₂SO₄ getrocknet und eingeengt. Die Reinigung des Rohproduktes erfolgte durch Chromatographie an 300 g Kieselgel mit einem Hexan-Essigester-Gemisch (20:1) als Laufmittel. Ausbeute: 54 % (farblosen Kristalle).

¹H-NMR (in CDCl₃, TMS als interner Standard): 2,3 ppm (s,3H; CH₃); 3,97 ppm (s,3H; OCH₃); 5,2 ppm (s,2H; CH2); 6,8-7,9 ppm (m,8H; aromat. Protonen).

### Beispiel 12 (Vergleichsbeispiele nach dem Stand der Technik)

### 12.1) Grignard-Reaktion von 2-[(2'-Methyl)-phenoxymethyl]-brombenzol mit Methyloxalylimidazol (vgl. EP-A 280 185)

110,8 g (0,4 mol) 2-[(2'-Methyl)-phenoxymethyl]-brombenzol wurden in 200 ml Tetrahydrofuran mit 10,4 g (0,4 mol) Magnesiumspänen umgesetzt. Anschließend tropfte man die erhaltene Grignard-Verbindung bei (-78)°C in eine Lösung von 61,6 g (0,4 mol) Methyloxalylimidazol in 500 ml Tetrahydrofuran. Nachdem sich die Mischung auf 20-25°C erwärmt hatte, wurde sie noch etwa 15 Stunden gerührt und dann auf 800 ml Eiswasser gegossen. Man extrahierte die Lösung dreimal mit je 200 ml Diethylether wonach die vereinigten Etherphasen neutral gewaschen, getrocknet und eingeengt wurden. Das Rohprodukt wurde durch Chromatographie an 190 g Kieselgel mit Cyclohexan/Essigsäureethylester (9:1) als Laufmittel gereinigt. Ausbeute: 37,8 %.

### 12.2) Umsetzung von 2-[2-Methylphenoxymethyl]-benzoylcyanid unter NaBr-Katalyse (vgl. US-A 4 234 739)

Zu einer Mischung aus 19,6 ml 85 gew.-%iger Schwefelsäure und 1,09 g (0,01 mol) Natriumbromid wurde bei 20-25°C eine Lösung von 20,5 g (81 mmol) 2-[2-Methylphenoxymethyl]-benzoylcyanid in 80 ml Methylenchlorid getropft. Danach versetzte man das Gemisch bei 40°C mit 38,9 ml (0,95 mol) Methanol und rührte noch 3 Stunden bei dieser Temperatur. Die HPLC-Analyse einer entnommenen Probe zeigte, daß nicht 2-[(2'-Methyl)-phenoxymethyl]-phenyl-glyoxylsäuremethylester, sondern quantitativ 2-[2-Methylphenoxymethyl]-benzoesäuremethylester entstanden war.

Nach Zugabe von 180 ml Wasser wurde die erhaltene Mischung dreimal mit je 50 ml Methylenchlorid extrahiert, wonach man die vereinigten organischen Phasen wie üblich auf das entstandene Produkt hin aufarbeitete.

¹H-NMR (in CDCl₃, TMS als interner Standard): 2,33 ppm (s,3H); 3,87 ppm (s,3H); 5,48 ppm (s,2H); 6,83-6,01 ppm (m,8H).

## Patentansprüche

1. Verfahren zur derstellung von E-Oximethern von Phenylglyoxylsäureestern der allgemeinen Formel I 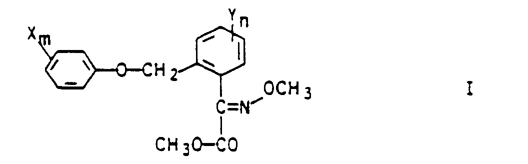 wobei die Variablen die folgende Bedeutung haben:
X,Y Substituenten ausgewählt aus einer Gruppe bestehend aus Halogen, C₁-C₄-Alkyl, C₁-C₄-Alkoxy oder Trifluormethyl,
m eine ganze Zahl von 0 bis 4,
n eine ganze Zahl von 0 bis 3,
dadurch gekennzeichnet, daß man ein o-Phenoxymethylbenzoylcyanid der allgemeinen Formel VI 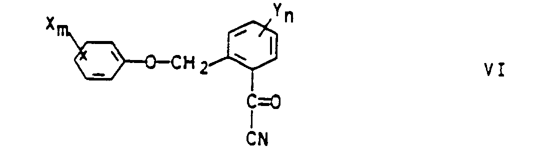 in Methanol in Gegenwart einer Säure in ein Gemisch enthaltend die Derivate VIIa und VIIb 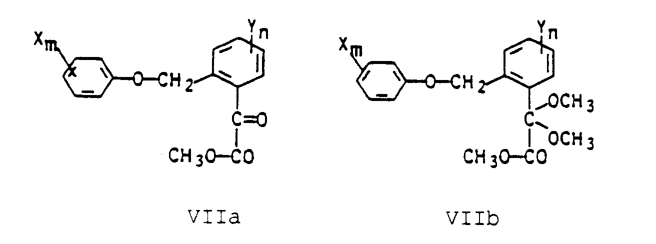 überführt und dieses Gemisch anschließend mit O-Methylhydroxylamin oder einem seiner Säureadditionssalze umsetzt und gleichzeitig oder anschließend mit einer Säure behandelt.

2. Verfahren gemäß Anspruch 1, dadurch gekennzeichnet, daß man ein o-Phenoxymethylbenzoylcyanid der allgemeinen Formel VI gemäß Anspruch 1 in Methanol in Gegenwart einer Säure umsetzt und die dabei als Nebenprodukte entstehenden Ketocarbonsäureester-dimethylketale der Formel VIIb durch Behandlung mit Säuren in die Verbindungen VIIa überführt.

3. Verfahren gemäß Anspruch 1, dadurch gekennzeichnet, daß man ein o-Phenoxymethylbenzoylcyanid der allgemeinen Formel VI gemäß Anspruch 1 in Methanol in Gegenwart einer Säure umsetzt und die dabei ebenfalls entstehenden Ketocarbonsäureester-dimethylketale der Formel VIIb unter sauren Bedingungen spaltet und gewüschtenfalls die als Nebenprodukte entstehenden α-Ketocarbonsäureamide der allgemeinen Formel VIIc 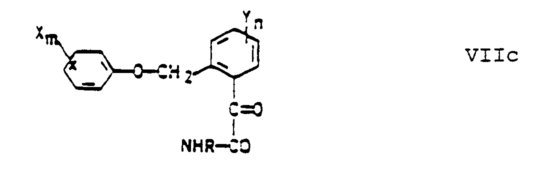 wobei R Wasserstoff oder die Acylgruppe bedeutet, erneut mit Methanol in Gegenwart einer Säure umsetzt.

4. Verfahren zur Herstellung eines Gemisches aus α-Ketocarbonsäureestern der allgemeinen Formel VIIa und Ketocarbonsäureester-dimethylketalen der allgemeinen Formel VIIb gemäß Anspruch 1, dadurch gekennzeichnet, daß man ein o-Phenoxymethylbenzoylcyanid der allgemeinen Formel VI gemäß Anspruch 1 in Methanol in Gegenwart einer Säure umsetzt.

5. Verwendung eines Gemisches aus α-Ketocarbonsäureestern der Formel VIIa und Ketocarbonsäureester-dimethylketalen der Formel VIIb gemäß Anspruch 1 zur Herstellung von Phenylglyoxylsäureestern der allgemeinen Formel I gemäß Anspruch 1.

6. Ketocarbonsäureester-dimethylketale der allgemeinen Formel VIIb gemäß Anspruch 1.

7. α-Ketocarbonsäureamide der allgemeinen Formel VIIc gemäß Anspruch 3.

## Claims

1. A process for preparing E-oxime ethers of phenylglyoxylic esters of the general formula I 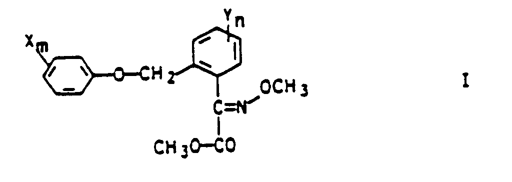 where the variables have the following meanings:
X, Y substituents selected from a group consisting of halogen, C₁-C₄-alkyl, C₁-C₄-alkoxy or trifluoromethyl,
m an integer from 0 to 4,
n an integer from 0 to 3,
which comprises converting an o-phenoxymethylbenzoyl cyanide of the general formula VI 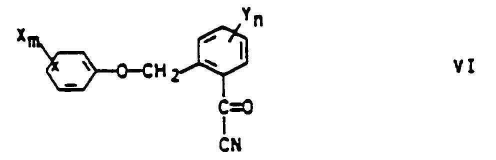 in methanol in the presence of an acid into a mixture containing the derivatives VIIa and VIIb 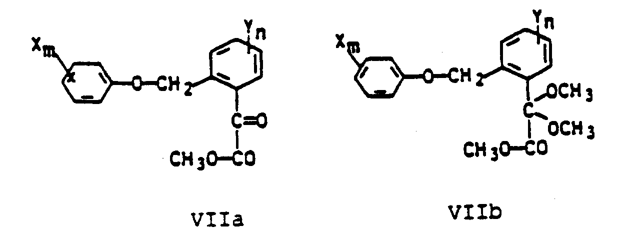 and subsequently reacting this mixture with O-methylhydroxylamine or one of its acid addition salts, and treating simultaneously or subsequently with an acid.

2. A process as claimed in claim 1, wherein an o-phenoxymethylbenzoyl cyanide of the general formula VI as set forth in claim 1 is reacted in methanol in the presence of an acid, and the keto carboxylic ester dimethyl ketals of the formula VIIb which are by-products thereof are converted by treatment with acids into the compounds VIIa.

3. A process as claimed in claim 1, wherein an o-phenoxymethylbenzoyl cyanide of the general formula VI as set forth in claim 1 is reacted in methanol in the presence of an acid, and the keto carboxylic ester dimethyl ketals of the formula VIIb which are also produced are cleaved under acidic conditions and, if required, the α-keto carboxamide by-products of the general formula VIIc 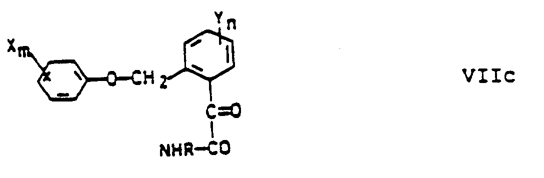 where R is hydrogen or the acyl group, are reacted anew with methanol in the presence of an acid.

4. A process for preparing a mixture of α-keto carboxylic esters of the general formula VIIa and keto carboxylic ester dimethyl ketals of the general formula VIIb as set forth in claim 1, which comprises reacting an o-phenoxymethylbenzoyl cyanide of the general formula VI as set forth in claim 1 in methanol in the presence of an acid.

5. The use of a mixture of α-keto carboxylic esters of the formula VIIa and keto carboxylic ester dimethyl ketals of the formula VIIb as set forth in claim 1 for preparing phenylglyoxylic esters of the general formula I as set forth in claim 1.

6. A keto carboxylic ester dimethyl ketal of the general formula VIIb as set forth in claim 1.

7. An α-keto carboxamide of the general formula VIIc as set forth in claim 3.

## Revendications

1. Procédé de préparation de E-oxyméthers d'esters d'acide phénylglyoxylique de formule générale I 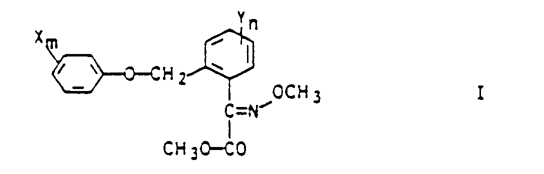 les variables ayant la signification suivante :
X, Y substituants choisis d'un groupe constitué d'halogène, alkyle en C1-C4, alcoxy en C1-C4 ou trifluorométhyle
m un nombre entier de 0 à 4
n un nombre entier de 0 à 3
caractérisé par le fait que l'on transforme un o-phénoxyméthylbenzoylyanure de la formule générale VI 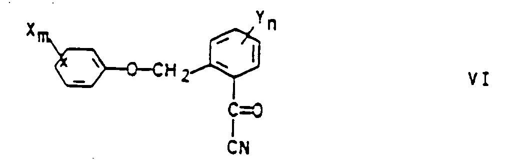 dans du méthanol en présence d'un acide, en un mélange contenant les dérivés VIIa et VIIb 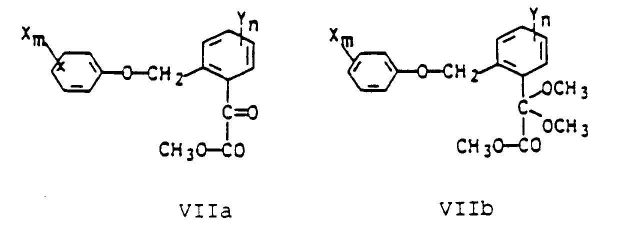 et on fait réagir ensuite un mélange avec O-méthylhydroxylamine ou un de ses sels d'addition d'acide et on le traite ensuite ou simultanément avec un acide.

2. Procédé selon la revendication 1, caractérisé par le fait que l'on fait réagir, dans du méthanol en présence d'un acide, un phénoxyméthylbenzoylcyanure de formule générale VI selon la revendication 1 et l'on transforme par traitement avec des acides les esters d'acide cétoncarboxylique-diméthylcétales ainsi obtenus comme produits secondaires, en les composés VIIa.

3. Procédé selon la revendication 1, caractérisé par le fait que l'on fait réagir, dans du méthanol en présence d'un acide, un phénoxyméthylbenzoylcyanure de formule générale VI selon la revendication 1 et l'on scinde, sous conditions acides, les esters d'acide cétoncarboxylique-diméthylcétales ainsi obtenus également de formule VIIb et, si on le souhaite on le fait réagir à nouveau avec du méthanol, en présence d'un acide, les amides d'acide α-cétocarboxylique obtenus comme produits secondaires et de formule générale : 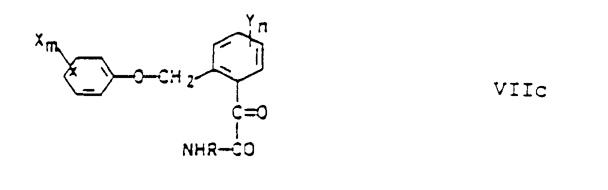 R représentant l'hydrogène ou le groupe acyle.

4. Procédé de préparation d'un mélange d'ester d'acide α-cétocarboxylique de formule générale VIIa et d'ester d'acide cétocarboxylique-diméthylcétales de formule générale VIIb selon la revendication 1, caractérisé par le fait que l'on fait réagir, dans le méthanol en présence d'un acide, un o-phénoxyméthylbenzoylcyanure de formule générale VI selon la revendication 1.

5. Utilisation d'un mélange d'ester d'acide α-cétocarboxylique de formule générale VIIa et d'ester d'acide cétocarboxylique-diméthylcétales de la formfule générale VIIb selon la revendication 1 pour la préparation d'esters d'acide phénylglyoxylique de formule générale I selon la revendication 1.

6. Ester d'acide cétocarboxylique-diméthylacétale de formule générale VIIb selon la revendication 1.

7. Amide d'acide α-cétocarboxylique de formule générale VIIc selon la revendication 3.
